(19) Europäisches Patentamt
European Patent Office
Office européen des brevets

(11) Veröffentlichungsnummer: **0 477 646 B1**

(12) # EUROPÄISCHE PATENTSCHRIFT

(45) Veröffentlichungstag der Patentschrift: **01.03.95**

(51) Int. Cl.[6]: **C07D 249/12**, C07D 401/12, C07D 413/12, C07D 417/12, A01N 43/653

(21) Anmeldenummer: **91115237.9**

(22) Anmeldetag: **10.09.91**

Die Akte enthält technische Angaben, die nach dem Eingang der Anmeldung eingereicht wurden und die nicht in dieser Patentschrift enthalten sind.

(54) **Substituierte 5-Alkoxy-1,2,4-triazol-3-(thi)one.**

(30) Priorität: **22.09.90 DE 4030063**

(43) Veröffentlichungstag der Anmeldung:
**01.04.92 Patentblatt 92/14**

(45) Bekanntmachung des Hinweises auf die Patenterteilung:
**01.03.95 Patentblatt 95/09**

(84) Benannte Vertragsstaaten:
**BE CH DE DK ES FR GB IT LI NL**

(56) Entgegenhaltungen:
**EP-A- 0 267 491**
**EP-A- 0 283 876**
**EP-A- 0 298 371**
**EP-A- 0 422 469**

**CHEMICAL ABSTRACTS, Band 101, Nr. 9, 27. August 1984, Columbus, Ohio, USA CHENG, CHEN CHIH et al. "Reaction of triazolinediones with acetylenes. Electrophillic addition" Seite 579, Spalte 1, Zusammenfassung-Nr. 71 922s & J. Org. Chem. 1984, 49(16) 2917-22**

(73) Patentinhaber: **BAYER AG**
**D-51368 Leverkusen (DE)**

(72) Erfinder: **Müller, Klaus-Helmut, Dr.**
**Bockhackstrasse 55**
**W-4000 Düsseldorf 13 (DE)**
Erfinder: **König, Klaus, Dr.**
**Zum Hahnberg 40**
**W-5068 Odenthal (DE)**
Erfinder: **Kluth, Joachim, Dr.**
**Tannenweg 9**
**W-4019 Langenfeld (DE)**
Erfinder: **Lürssen, Klaus, Dr.**
**August-Kierspel-Strasse 145**
**W-5060 Bergisch Gladbach 2 (DE)**
Erfinder: **Santel, Hans-Joachim, Dr.**
**Grünstrasse 9a**
**W-5090 Leverkusen (DE)**
Erfinder: **Schmidt, Robert R., Dr.**
**Im Waldwinkel 110**
**W-5060 Bergisch Gladbach 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**CHEMICAL ABSTRACTS, Band 89, Nr. 3, 17. Juli 1978, Columbus, Ohio, USA ALTLAND,HENRY W. et al. "Cyclization of 1-substituted-3-thiosemicarbazides to triazole derivatives under alkaline conditions" Seite 644, Spalte 2, Zusammenfassung-Nr. 24 226s & J. Heterocycl. Chem. 1978, 15 (3), 377-84**

**CHEMICAL ABSTRACTS, Band 68, Nr. 7, 12. Februar 1968, Columbus, Ohio, USA R.CLARKSON et al. "2-Alkoxy-5-amino- and -5-arenesulfonamido-1,3,4- -thiadiazolesand related compounds" Seite 2875, Spalte 2, Zu- sammenfassung-Nr. 29 639e & J. Chem. Soc., C 1967(24), 2700-4**

## Beschreibung

Die Erfindung betrifft neue substituierte 5-Alkoxy-1,2,4-triazol-3-(thi)one, mehrere Verfahren sowie neue Zwischenprodukte zu ihrer Herstellung und ihre Verwendung als Herbizide.

Es ist bekannt, daß bestimmte substituierte Triazolone, wie z.B. 4-Amino-5-methyl-2-phenylaminocarbonyl-2,4-dihydro-3H-1,2,4-triazol-3-on, herbizide Eigenschaften aufweisen (vgl. EP-A 283876, EP-A 294666, EP-A 298371). Die herbizide Wirksamkeit dieser Verbindungen ist jedoch nicht in allen Anwendungsgebieten völlig zufriedenstellend.

Es wurden nun neue substituierte 5-Alkoxy-1,2,4-triazol-3-(thi)one der allgemeinen Formel (I)

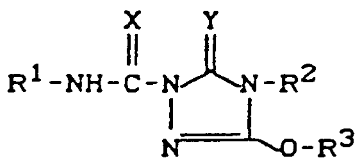

(I)

in welcher

$R^1$ für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als

Substituenten im cyclischen und gegebenenfalls im aliphatischen Teil ausgewählt sind: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem $R^1$ für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als

Substituenten ausgewählt sind: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem $R^1$ für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aryloxyalkyl, Arylalkenyl, Arylalkinyl, Aralkyloxy, Aryloxy, Aroyl oder Aryl mit jeweils 6 oder 10 Kohlenstoffatomen im Arylteil und gegebenenfalls bis zu 8 Kohlenstoffatomen im Alkyl-, Alkenyl- oder Alkinylteil steht, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl

oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffatomen steht,

X für Sauerstoff oder Schwefel steht und

Y für Sauerstoff oder Schwefel steht

und Halogen in den in $R^1$, $R^2$ und $R^3$ genannten Substitutionsmustern für Fluor, Chlor, Brom und Iod steht, gefunden.

Weiter wurde gefunden, daß man die neuen substituierten 5-Alkoxy-1,2,4-triazol-3-(thi)one der allgemeinen Formel (I) erhält, wenn man

(a) 5-Alkoxy-1,2,4-triazol-3-(thi)one der allgemeinen Formel (II)

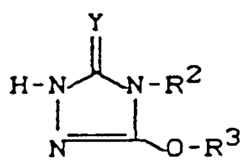

(II)

in welcher

$R^2$, $R^3$ und Y die oben angegebene Bedeutung haben,

mit Iso(thio)cyanaten der allgemeinen Formel (III)

$R^1$-N=C=X (III)

in welcher

$R^1$ und X die oben angegebene Bedeutung haben,

gegebenenfalls in Gegenwart eines Verdünnungsmittels und gegebenenfalls in Gegenwart eines basischen Reaktionshilfsmittels umsetzt, oder wenn man

(b) 5-Alkoxy-1,2,4-triazol-3-(thi)one der allgemeinen Formel (II)

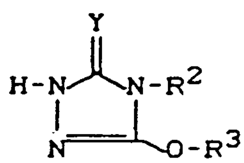

(II)

in welcher

$R^2$, $R^3$ und Y die oben angegebene Bedeutung haben,

mit reaktionsfähigen (Thio-)Carbamaten der allgemeinen Formel (IV)

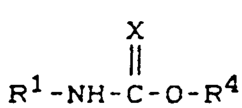

(IV)

in welcher

$R^1$ und X die oben angegebene Bedeutung haben und

$R^4$ für Alkyl, Aralkyl oder Aryl steht,

gegebenenfalls in Gegenwart eines Verdünnungsmittels und gegebenenfalls in Gegenwart eines basischen Reaktionshilfsmittels umsetzt, oder wenn man

(c) 5-Alkoxy-1,2,4-triazol-3-(thi)on-Derivate der allgemeinen Formel (V)

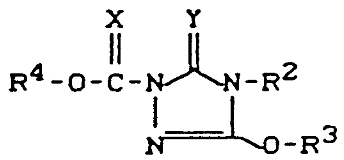

(V)

in welcher

$R^2$, $R^3$, X und Y die oben angegebene Bedeutung haben und

$R^4$ für Alkyl, Aralkyl oder Aryl steht,

mit Aminoverbindungen der allgemeinen Formel (VI)

$R^1$-$NH_2$ (VI)

in welcher

$R^1$ die oben angegebene Bedeutung hat,

gegebenenfalls in Gegenwart eines Verdünnungsmittels und gegebenenfalls in Gegenwart eines basischen Reaktionshilfsmittels umsetzt.

Schließlich wurde gefunden, daß die neuen substituierten 5-Alkoxy-1,2,4-triazol-3-(thi)one der allgemeinen Formel (I) sich durch starke herbizide Wirksamkeit auszeichnen.

Überraschenderweise zeigen die neuen Verbindungen der Formel (I) erheblich bessere herbizide Wirkung als die bekannte Verbindung 4-Amino-5-methyl-2-phenylaminocarbonyl-2,4-dihydro-3H-1,2,4-triazol-3-on.

Die erfindungsgemäßen substituierten 5-Alkoxy-1,2,4-triazol-3-(thi)one sind durch die Formel (I) allgemein definiert. Bevorzugt sind Verbindungen der Formel (I), bei welchen

$R^1$ für Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Dodecyl, für Allyl, Propenyl, n- oder i-Butenyl, n- oder i-Pentenyl, n- oder i-Hexenyl, Propargyl, n- oder i-Butinyl, n-oder i-Pentinyl, n- oder i-Hexinyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 4 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein-bis dreifach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclopentylmethyl, Cyclohexylmethyl, Cyclohexylethyl, Cyclohexenyl oder Cyclohexenylmethyl stehen, wobei als besonders bevorzugte Substituenten im cyclischen und gegebenenfalls im aliphatischen Teil ausgewählt sind: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl; außerdem $R^1$ für im Heterocyclylteil gegebenenfalls ein- bis dreifach, gleich oder verschieden substituiertes Heterocyclylmethyl, Heterocyclylethyl oder Heterocyclylpropyl steht, wobei als Heterocyclen jeweils in Frage kommen:

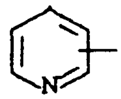 ; 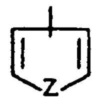 ; 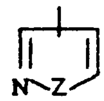 ; 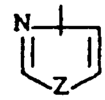 ;

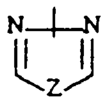 ; 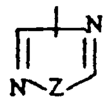 ; 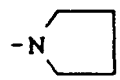 ; 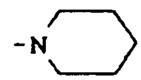 ;

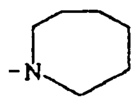 ; 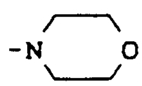 oder 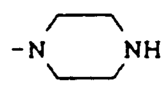

wobei Z jeweils für Sauerstoff oder Schwefel steht und wobei als besonders bevorzugte Substituenten ausgewählt sind:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem $R^1$ für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Phenoxymethyl, Phenoxyethyl, Phenoxypropyl, Phenoxybutyl, Phenylethinyl, Benzyloxy, Phenylethyloxy, Phenoxy, Benzoyl, Phenyl oder Naphthyl steht, wobei als Phenylsubstituenten jeweils in Frage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Difluormethoxy, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl und Phenoxy,

$R^2$ für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl,Halogenalkenyl und Halogenalkinyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclopentylmethyl Cyclohexylmethyl oder Cyclohexylethyl steht,

$R^3$ für Methyl, Ethyl, n- oder i-Propyl, n-, i-, s- oder t-Butyl, n-, i-, s- oder t-Pentyl, n- oder i-Hexyl, für Allyl, Propargyl, Methoxyethyl, Ethoxyethyl, Fluorethyl, Chlorethyl, Fluorpropyl, Chlorpropyl oder Cyanoethyl steht,

X für Sauerstoff oder Schwefel steht und

Y für Sauerstoff oder Schwefel steht.

Ganz besonders bevorzugt sind Verbindungen der Formel (I), bei welchen

$R^1$ für Methyl, Ethyl, n- oder i-Propyl, n-, i-, s- oder t-Butyl, n-, i-, s- oder t-Pentyl, n-, i-, s- oder t-Hexyl, Propargyl, n- oder i-Butinyl, n- oder i-Pentinyl, für geradkettiges oder verzweigtes Halogenalkyl mit 1 bis 4 Kohlenstoffatomen und 1 bis 4 gleichen oder verschiedenen Halogenatomen, insbesondere Fluor und Chlor, für Cyanomethyl, Cyanethyl, n- oder i-Cyanopropyl, für jeweils gegebenenfalls einfach bis dreifach, gleich oder verschieden durch Fluor, Chlor, Brom, Methyl und/oder Ethyl substituiertes Cyclopropyl, Cyclopropylmethyl, Cyclopentyl, Cyclopentylmethyl, Cyclohexyl oder Cyclohexylmethyl, oder für jeweils gegebenenfalls einfach bis dreifach, gleich oder verschieden durch Fluor, Chlor, Brom, Methyl, Ethyl und/oder Trifluormethyl substituiertes Phenyl, Benzyl, Phenylethyl, jeweils geradkettiges oder verzweigtes Phenylpropyl, Phenylbutyl, Phenylpentyl, Phenylhexyl oder für Morpholinyl-$C_1$-$C_4$-alkyl steht,

$R^2$ für Methyl, Ethyl, n-, i- oder Cyclopropyl, n-, i-, s-oder t-Butyl steht,

$R^3$ für Methyl, Ethyl, n- oder i-Propyl, n-, i-, s- oder t-Butyl, n- oder i-Pentyl steht,

X für Sauerstoff steht und

Y für Sauerstoff steht.

Beispiele für die erfindungsgemäßen Verbindungen der Formel (I) sind in der nachstehenden Tabelle 1 aufgeführt.

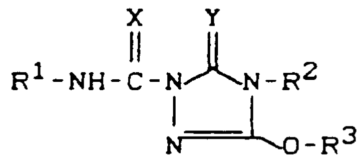

(I)

Tabelle 1: Beispiele für die Verbindungen der Formel (I)

| $R^1$ | $R^2$ | $R^3$ | X | Y |
|---|---|---|---|---|
| $i\text{-}C_3H_7$ | $CH_3$ | $CH_3$ | O | O |
| $CF_3\text{-}C(CH_3)_2\text{-}$ | $CH_3$ | $C_2H_5$ | O | O |
| $F\text{-}CH_2\text{-}C(CH_3)_2\text{-}$ | $CH_3$ | $C_2H_5$ | O | O |
| $C_2H_5\text{-}C(CH_3)_2\text{-}$ | $CH_3$ | $CH_3$ | O | O |
| $C_3H_7\text{-}C(CH_3)_2\text{-}$ | $CH_3$ | $CH_3$ | O | O |
| $i\text{-}C_3H_7\text{-}C(CH_3)_2\text{-}$ | $CH_3$ | $CH_3$ | O | O |
| 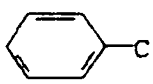 $C(CH_3)_2\text{-}$ | $CH_3$ | $CH_3$ | O | O |
| 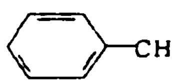 $CH_2\text{-}C(CH_3)_2\text{-}$ | $CH_3$ | $CH_3$ | O | O |

Tabelle 1 - Fortsetzung

| $R^1$ | $R^2$ | $R^3$ | X | Y |
|---|---|---|---|---|
| 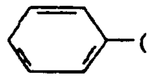$-(CH_2)_2-C(CH_3)_2-$ | $CH_3$ | $CH_3$ | O | O |
| 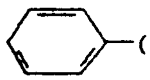$-(CH_2)_2-C(CH_3)_2-$ | $CH_3$ | $CH_3$ | O | O |
| 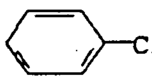$-CH_2-C(CH_3)_2-$ | $CH_3$ | $CH_3$ | O | O |
| 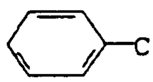$-CH(CH_3)-$ | $CH_3$ | $CH_3$ | O | O |
| 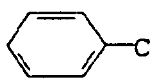$-C(CH_3)_2-$ | $CH_3$ | $C_2H_5$ | O | O |
| $CH_3O$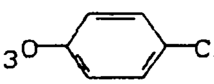$-CH_2-CH_2-C(CH_3)_2-$ | $CH_3$ | $CH_3$ | O | O |

Verwendet man beispielsweise 5-Ethoxy-4-ethyl-2,4-dihydro-3H-1,2,4-triazol-3-thion und Isopropylisocyanat als Ausgangsstoffe, so läßt sich der Reaktionsablauf des erfindungsgemäßen Verfahrens (a) durch das folgende Formelschema darstellen:

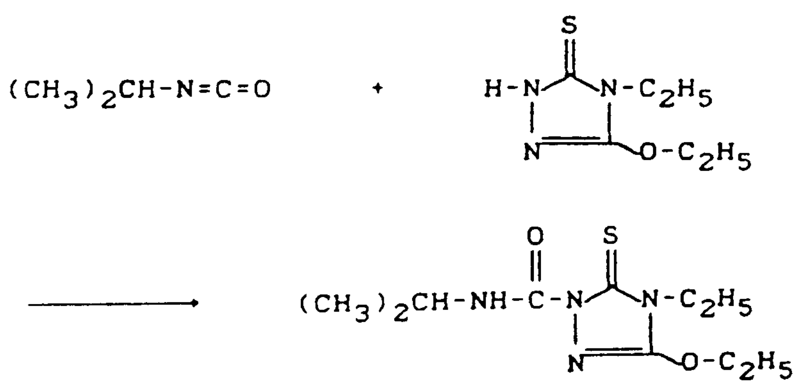

Verwendet man beispielsweise 5-Isopropoxy-4-methyl-2,4-dihydro-3H-1,2,4-triazol-3-on und N-tert-Butyl-O-methylthiocarbamat als Ausgangsstoffe, so läßt sich der Reaktionsablauf des erfindungsgemäßen Verfahrens (b) durch das folgende Formelschema darstellen:

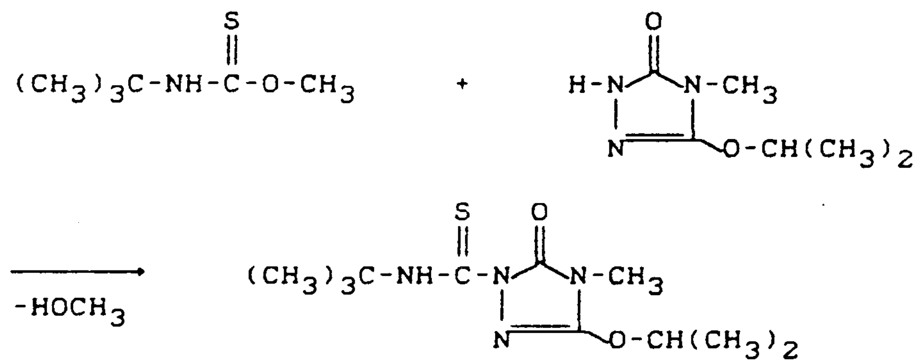


Verwendet man beispielsweise 4-Isopropyl-5-methoxy-2-phenoxycarbonyl-2,4-dihydro-3H-1,2,4-triazol-3-on und Cyclohexylamin als Ausgangsstoffe, so läßt sich der Reaktionsablauf des erfindungsgemäßen Verfahrens (c) durch das folgende Formelschema darstellen:

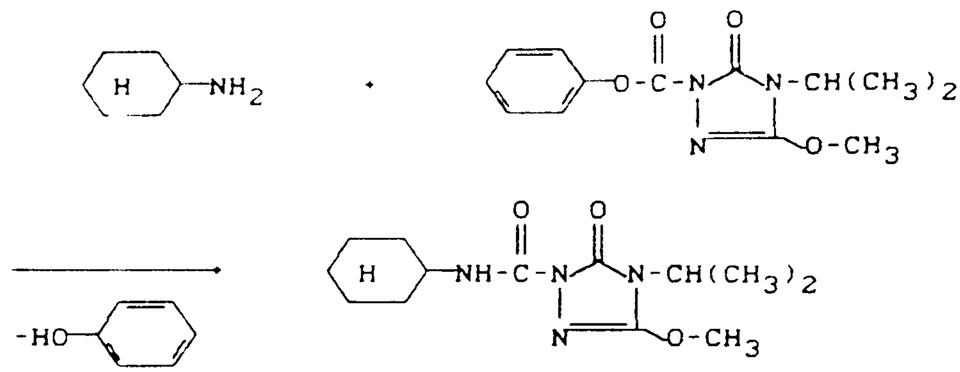

Die bei den erfindungsgemäßen Verfahren (a) und (b) zur Herstellung von Verbindungen der Formel (I) als Ausgangsstoffe zu verwendenden 5-Alkoxy-1,2,4-triazol-3-(thi)one sind durch die Formel (II) allgemein definiert.

In Formel (II) haben $R^2$, $R^3$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, $R^3$ und Y angegeben wurden.

Beispiele für die Ausgangsstoffe der Formel (II) sind in der nachstehenden Tabelle 2 aufgeführt.

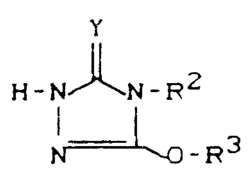

(II)

Tabelle 2

| Beispiele für die Ausgangsstoffe der Formel (II) | | |
|---|---|---|
| $R^2$ | $R^3$ | Y |
| $CH_3$ | $CH_3$ | O |
| $CH_3$ | $CH_3$ | S |
| $CH_3$ | $C_2H_5$ | O |
| $CH_3$ | $C_2H_5$ | S |
| $CH_3$ | $C_3H_7$ | O |
| $CH_3$ | $C_3H_7$ | S |
| $CH_3$ | $CH(CH_3)_2$ | O |
| $CH_3$ | $CH(CH_3)_2$ | S |
| $CH_3$ | $-CH_2-CH=CH_2$ | O |
| $CH_3$ | $-CH_2-CH=CH_2$ | S |
| $C_2H_5$ | $CH_3$ | O |
| $C_2H_5$ | $CH_3$ | S |
| $C_2H_5$ | $C_2H_5$ | O |
| $C_2H_5$ | $C_2H_5$ | S |
| $C_2H_5$ | $CH(CH_3)_2$ | O |
| $C_2H_5$ | $-CH_2-CH=CH_2$ | O |
| $C_2H_5$ | $CH(CH_3)_2$ | S |
| $C_2H_5$ | $-CH_2-CH=CH_2$ | S |

Die Ausgangsstoffe der Formel (II) sind teilweise bekannt (vgl. J. Chem. Soc C 1967, 2700-2704; J. Heterocycl. Chem. 15 (1978), 377-384).

Neu und Gegenstand der vorliegenden Patentanmeldung sind diejenigen Verbindungen der allgemeinen Formel (II), bei welchen Y für Sauerstoff steht und $R^2$ und $R^3$ die oben angegebenen Bedeutungen haben.

Man erhält die neuen 5-Alkoxy-1,2,4-triazol-3-one (II, Y = O), wenn man Hydrazinoameisensäureester der allgemeinen Formel (VII)

$H_2N-NH-CO-O-R^4$ (VII)

in welcher
$R^4$ die oben angegebene Bedeutung hat,
mit Alkyliminokohlensäurediestern der allgemeinen Formel (VIII)

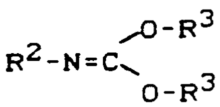 (VIII)

in welcher
$R^2$ und $R^3$ die oben angegebene Bedeutung haben,
gegebenenfalls in Gegenwart eines Verdünnungsmittels, wie z.B. o-Dichlorbenzol, bei Temperaturen zwischen 0°C und 200°C, vorzugsweise zwischen 50°C und 150°C umsetzt (vgl. die Herstellungsbeispiele).

Die Ausgangsstoffe der Formeln (VII) und (VIII) sind bekannte Chemikalien.

Die beim erfindungsgemäßen Verfahren (a) zur Herstellung von Verbindungen der Formel (I) als Ausgangsstoffe zu verwendenden Iso(thio)cyanate sind durch die Formel (III) allgemein definiert.

In Formel (III) haben $R^1$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und X angegeben wurden.

Die Ausgangsstoffe der Formel (III) sind bekannte organische Synthesechemikalien.

Die beim erfindungsgemäßen Verfahren (b) zur Herstellung von Verbindungen der Formel (I) als Ausgangsstoffe zu verwendenden reaktionsfähigen (Thio)Carbamate sind durch die Formel (IV) allgemein definiert.

In Formel (IV) haben $R^1$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und X angegeben wurden;

$R^4$ steht vorzugsweise für Methyl, Ethyl, Phenyl oder Benzyl.

Die Ausgangsstoffe der Formel (IV) sind bekannte organische Synthesechemikalien.

Die beim erfindungsgemäßen Verfahren (c) zur Herstellung von Verbindungen der Formel (I) als Ausgangsstoffe zu verwendenden 5-Alkoxy-1,2,4-triazol-3-(thi)on-Derivate sind durch die Formel (V) allgemein definiert.

In Formel (V) haben $R^2$, $R^3$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, $R^3$, X und Y angegeben wurden;

$R^4$ steht vorzugsweise für Methyl, Ethyl, Phenyl oder Benzyl.

Die Ausgangsstoffe der Formel (V) sind noch nicht aus der Literatur bekannt und sind Gegenstand der vorliegenden Patentanmeldung.

Man erhält die neuen 5-Alkoxy-1,2,4-triazol-3-(thi)on-Derivate der allgemeinen Formel (V), wenn man 5-Alkoxy-1,2,4-triazol-3-(thi)one der allgemeinen Formel (II)

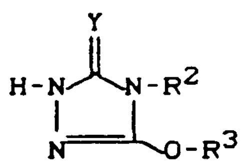

(II)

in welcher

$R^2$, $R^3$ und Y die oben angegebene Bedeutung haben,

mit Chlor(thio)ameisensäureestern der allgemeinen Formel (IX)

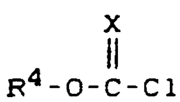

(IX)

in welcher

$R^4$ und X die oben angegebene Bedeutung haben,

gegebenenfalls in Gegenwart eines Verdünnungsmittels, wie z.B. Tetrahydrofuran, und gegebenenfalls in Gegenwart eines Säureakzeptors, wie z.B. Kalium-tert-butylat, bei Temperaturen zwischen -20 °C und +100 °C umsetzt.

Die beim erfindungsgemäßen Verfahren (c) zur Herstellung von Verbindungen der Formel (I) weiter als Ausgangsstoffe zu verwendenden Aminoverbindungen sind durch die Formel (VI) allgemein definiert.

In Formel (VI) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde.

Die Ausgangsstoffe der Formel (VI) sind bekannte organische Synthesechemikalien.

Die erfindungsgemäßen Verfahren (a), (b) und (c) zur Herstellung der neuen Verbindungen der Formel (I) werden jeweils vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die erfindungsgemäßen Verfahren (a), (b) und (c) werden jeweils gegebenenfalls in Gegenwart eines basischen Reaktionshilfsmittels durchgeführt. Als solche werden vorzugsweise basische organische Stickstoffverbindungen eingesetzt. Hierzu gehören beispielsweise Trimethylamin, Triethylamin, Tripropylamin,

Tributylamin, Diisopropylamin, Dicyclohexylamin, N,N-Dimethylanilin, N,N-Dimethylbenzylamin, Pyridin, 4-Dimethylamino-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b) und (c) jeweils in einem größeren Bereich variiert werden. Im allgemeinen arbeitet man bei Temperaturen zwischen -20 °C und +150 °C, vorzugsweise bei Temperaturen zwischen 0 °C und 100 °C.

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b) und (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines basischen Reaktionshilfsmittels durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern vor allem in monokotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); Methyl-4-aminobenzolsulfonylcarbamat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); 3,5-Dibrom-4-hydroxybenzonitril (BROMOXYNIL); N-(Butoxymethyl)-2-chlor-N-(2,6-diethylphenyl)-acetamid(BUTACHLOR); 5-Amino-4-chlor-2-phenyl-2,3-dihydro-3-oxy-pyridazin (CHLORIDAZON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); 1-Methyl-3-phenyl-5-(3-trifluormethylphenyl)-4-pyridon(FLURIDONE); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 10 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

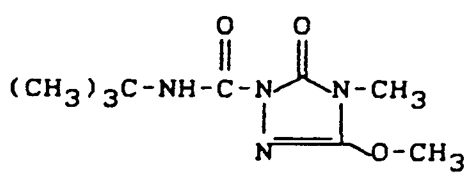

Eine Mischung aus 1,3 g (0,01 Mol) 5-Methoxy-4-methyl-2,4-dihydro-3H-1,2,4-triazol-3-on, 0,1 g 1,8-Diazabicyclo-[5,4,0]-undec-7-en (DBU), 1,0 g (0,01 Mol) tert-Butylisocyanat und 50 ml Acetonitril wird 12 Stunden bei 20 °C gerührt und anschließend im Wasserstrahlvakuum eingeengt. Der Rückstand wird in Methylenchlorid aufgenommen, mit Wasser gewaschen, mit Magnesiumsulfat getrocknet und filtriert. Das Filtrat wird im Wasserstrahlvakuum eingengt, der Rückstand durch Verreiben mit Petrolether zur Kristallisation gebracht und durch Absaugen isoliert.

Man erhält 1,4 g (61% der Theorie) 2-tert-Butyl-aminocarbonyl-5-methoxy-4-methyl-2,4-dihydro-3H-1,2,4-triazol-3-on vom Schmelzpunkt 167 °C.

Analog Beispiel 1 und entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

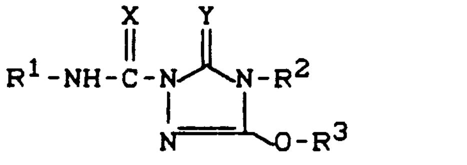

(I)

<u>Tabelle 3:</u> Herstellungsbeispiele für die Verbindungen der Formel (I)

| Bsp.-Nr. | $R^1$ | $R^2$ | $R^3$ | X | Y | Schmelzpunkt (°C) |
|---|---|---|---|---|---|---|
| 2 | 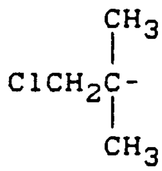 | $CH_3$ | $CH_3$ | O | O | 153 |
| 3 | 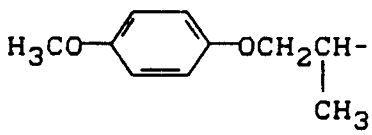 | $C_3H_7$ | $CH_3$ | O | O | (Öl) |
| 4 | 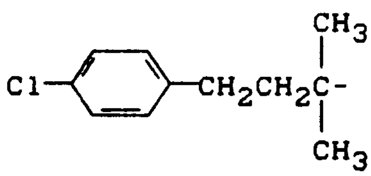 | $C_3H_7$ | $CH_3$ | O | O | (Öl) |

Tabelle 3 Fortsetzung

| Bsp.-Nr. | $R^1$ | $R^2$ | $R^3$ | X | Y | Schmelzpunkt (°C) |
|---|---|---|---|---|---|---|
| 5 | 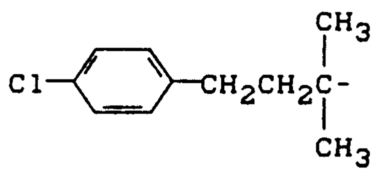 | $CH_3$ | $C_2H_5$ | O | O | (Öl) |
| 6 | 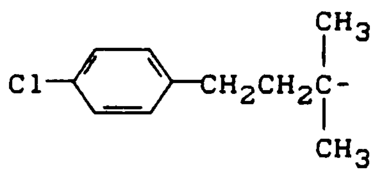 | $C_2H_5$ | $CH_3$ | O | O | (Öl) |
| 7 | 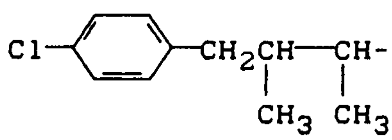 | $CH_3$ | $C_2H_5$ | O | O | (Öl) |
| 8 | 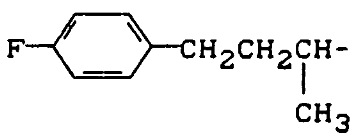 | $CH_3$ | $C_2H_5$ | O | O | (Öl) |
| 9 | 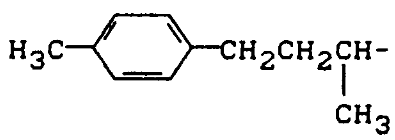 | $CH_3$ | $C_2H_5$ | O | O | (Öl) |
| 10 | 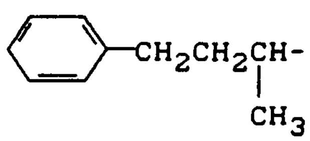 | $CH_3$ | $C_2H_5$ | O | O | (Öl) |

Tabelle 3 Fortsetzung

| Bsp.-Nr. | $R^1$ | $R^2$ | $R^3$ | X | Y | Schmelzpunkt (°C) |
|---|---|---|---|---|---|---|
| 11 | 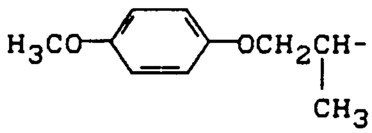 | $CH_3$ | $C_2H_5$ | O | O | (Öl) |
| 12 | 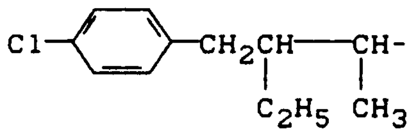 | $CH_3$ | $C_2H_5$ | O | O | (Öl) |
| 13 | 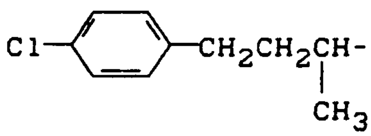 | $CH_3$ | $C_2H_5$ | O | O | (Öl) |
| 14 | 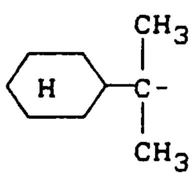 | $CH_3$ | $CH_3$ | O | O | 166 |
| 15 | 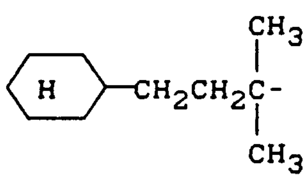 | $CH_3$ | $CH_3$ | O | O | 120 |
| 16 | 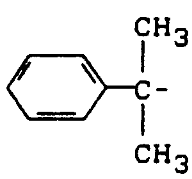 | $CH_3$ | $CH_3$ | O | O | 176 |

Tabelle 3 Fortsetzung

| Bsp.-Nr. | $R^1$ | $R^2$ | $R^3$ | X | Y | Schmelzpunkt (°C) |
|---|---|---|---|---|---|---|
| 17 | 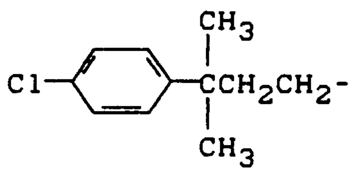 | $CH_3$ | $CH_3$ | O | O | 130 |
| 18 | 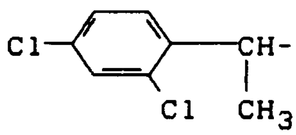 | $CH_3$ | $CH_3$ | O | O | 104 |
| 19 | 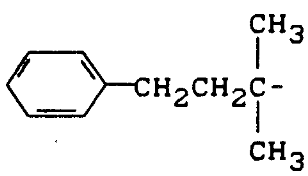 | $CH_3$ | $CH_3$ | O | O | 117 |
| 20 | 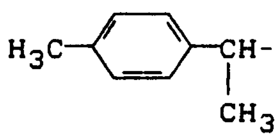 | $CH_3$ | $CH_3$ | O | O | 100 |
| 21 | $(CH_3)_3C-$ | $CH_3$ | $C_3H_7$ | O | O | 75 |
| 22 | 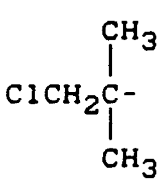 | $CH_3$ | $C_3H_7$ | O | O | 95 |

Tabelle 3 Fortsetzung

| Bsp.-Nr. | $R^1$ | $R^2$ | $R^3$ | X | Y | Schmelzpunkt (°C) |
|---|---|---|---|---|---|---|
| 23 | 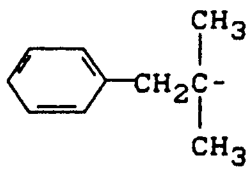 | $CH_3$ | $CH_3$ | O | O | 142 |
| 24 | 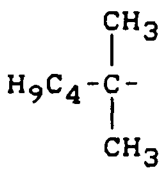 | $CH_3$ | $CH_3$ | O | O | 135 |
| 25 | 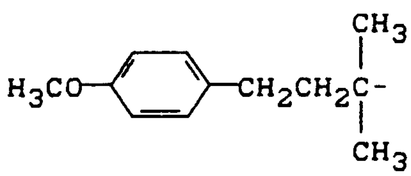 | $CH_3$ | $CH_3$ | O | O | 106 |
| 26 | 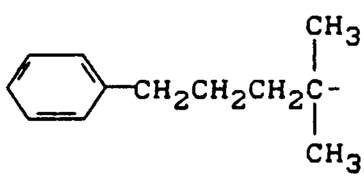 | $CH_3$ | $CH_3$ | O | O | 137 |
| 27 | 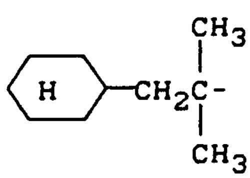 | $CH_3$ | $CH_3$ | O | O | 131 |
| 28 | 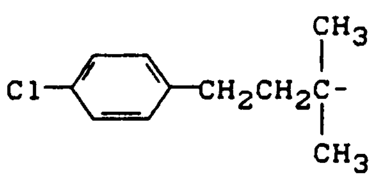 | $CH_3$ | $CH_3$ | O | O | 118 |

Tabelle 3 Fortsetzung

| Bsp.-Nr. | $R^1$ | $R^2$ | $R^3$ | X | Y | Schmelzpunkt (°C) |
|---|---|---|---|---|---|---|
| 29 | 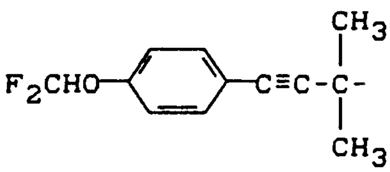 | $CH_3$ | $CH_3$ | O | O | 82 |
| 30 | $(CH_3)_3C-$ | $CH_2-CH=CH_2$ | $CH_3$ | O | O | 87 |

Ausgangsstoffe der Formel (II):

Beispiel (II-1)

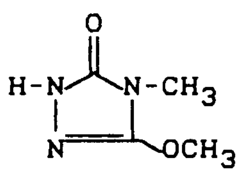

50,2 g (0,33 mol) Hydrazinoameisensäurephenylester und 36,6 g (0,33 mol, 93%ig) Trimethyliminocarbonat werden in 100 ml absolutem o-Dichlorbenzol auf 60 °C erwärmt und 2 Stunden gerührt, wobei eine klare Lösung entsteht. Im Verlauf von zwei Stunden wird auf 120 °C erwärmt, wobei Methanol abdestilliert. Es wird vorsichtig Vakuum angelegt, wobei weiteres Methanol und zuletzt Phenol abdestilliert. Bei weiterem Destillieren entsteht eine Fraktion, die in der Vorlage kristallin erstarrt.

Nach Umkristallisieren aus Toluol werden 7,0 g (0,054 Mol, 16% der Theorie) 4-Methyl-5-methoxy-2,4-dihydro-3H-1,2,4-triazol-3-on in Form farbloser Kristalle vom Schmelzpunkt 142-144 °C erhalten.

Anwendungsbeispiele:

In den Anwendungsbeispielen wird die folgende Verbindung (A) als Vergleichssubstanz herangezogen:

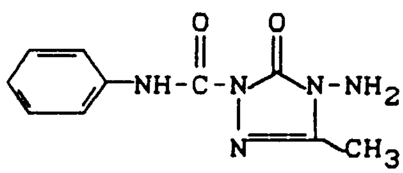

(A)

4-Amino-5-methyl-2-phenylaminocarbonyl-2,4-dihydro-3H-1,2,4-triazol-3-on
(bekannt aus EP-A 294666).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 1 und 2.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt beispielsweise die Verbindung gemäß Herstellungsbeispiel 2 bei guter Verträglichkeit gegenüber Kulturpflanzen, wie z.B. Gerste, starke Wirkung gegen Unkräuter.

## Patentansprüche

**Patentansprüche für folgende Vertragsstaaten : BE, CH, DE, DK, FR, GB, IT, LI, NL**

**1.** Substituierte 5-Alkoxy-1,2,4-triazol-3-(thi)-one der allgemeinen Formel (I)

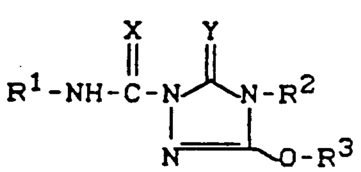

(I)

in welcher

$R^1$ für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten im cyclischen und gegebenenfalls im aliphatischen Teil ausgewählt sind: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem $R^1$ für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen -insbesondere Stickstoff, Sauerstoff und/oder Schwefel - im Heterocyclylteil steht, wobei als Substituenten ausgewählt sind: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem $R^1$ für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aryloxyalkyl, Arylalkenyl, Arylalkinyl, Aralkyloxy, Aryloxy, Aroyl oder Aryl mit jeweils 6 oder 10 Kohlenstoffatmen im Arylteil und gegebenfalls bis zu 8 Kohlenstoffatomen im Alkyl-, Alkenyl- oder Alkinylteil steht, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alxoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffatomen steht,

X für Sauerstoff oder Schwefel steht und

Y für Sauerstoff oder Schwefel steht

und Halogen in den in $R^1$, $R^2$ und $R^3$ genannten Substitutionsmustern für Fluor, Chlor, Brom und Iod steht.

**2.** Substituierte 5-Alkoxy-1,2,4-triazol-3-(thi)one der allgemeinen Formel (I) gemäß Anspruch 1, bei welchen

$R^1$ für Methyl, Ethyl, n- oder i-Propyl, n-, i-, s-oder t-Butyl, jeweils geradkettiges oder verzweigtes Pentyl, Hexyl, Heptyl, Octyl, Nonyl, Decyl oder Dodecyl, für Allyl, Propenyl, n- oder i-Butenyl, n- oder i-Pentenyl, n- oder i-Hexenyl, Propargyl, n-oder i-Butinyl, n-oder i-Pentinyl, n- oder i-Hexinyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit

jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 4 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclopentylmethyl, Cyclohexylmethyl, Cyclohexylethyl, Cyclohexenyl oder Cyclohexenylmethyl stehen, wobei als besonders bevorzugte Substituenten im cyclischen und gegebenenfalls im aliphatischen Teil ausgewählt sind: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl; außerdem $R^1$ für im Heterocyclylteil gegebenenfalls ein- bis dreifach, gleich oder verschieden substituiertes Heterocyclylmethyl, Heterocyclylethyl oder Heterocyclylpropyl steht, wobei als Heterocyclen jeweils in Frage kommen:

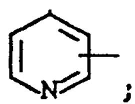 ; 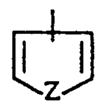 ; 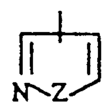 ; 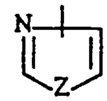 ;

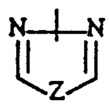 ; 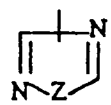 ; 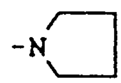 ; 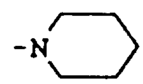 ;

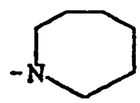 ; 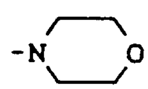 oder 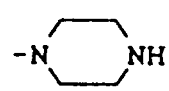

wobei Z jeweils für Sauerstoff oder Schwefel steht und wobei als besonders bevorzugte Substituenten ausgewählt sind:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem $R^1$ für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Phenoxymethyl, Phenoxymethyl, Phenoxypropyl, Phenoxybutyl, Phenylethinyl, Benzyloxy, Phenylethyloxy, Phenoxy, Benzoyl, Phenyl oder Naphthyl steht, wobei als Phenylsubstituenten jeweils in Frage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Difluormethoxy, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl und Phenoxy,

$R^2$ für Methyl, Ethyl, n- oder i-Propyl, n-, i-, s-oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl, Halogenalkenyl und Halogenalkinyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclopentylmethyl, Cyclohexylmethyl oder Cyclohexylethyl steht,

$R^3$ für Methyl, Ethyl, n- oder i-Propyl, n-, i-, s-oder t-Butyl, n-, i-, s- oder t-Pentyl, n- oder i-Hexyl, für Allyl, Propargyl, Methoxyethyl, Ethoxyethyl, Fluorethyl, Chlorethyl, Fluorpropyl, Chlorpropyl oder Cyanoethyl steht,

X für Sauerstoff oder Schwefel steht und

Y für Sauerstoff oder Schwefel steht.

**3.** Substituierte 5-Alkoxy-1,2,4-triazol-3-(thi)one der allgemeinen Formel (I) gemäß Anspruch 1, bei welchen

$R^1$ für Methyl, Ethyl, n- oder i-Propyl, n-, i-, s-oder t-Butyl, n-, i-, s- oder t-Pentyl, n-, i-, s-oder t-Hexyl, Propargyl, n- oder i-Butinyl, n- oder i-Pentinyl, für geradkettiges oder verzweigtes Halogenalkyl mit 1 bis 4 Kohlenstoffatomen und 1 bis 4 gleichen oder verschiedenen Halogenatomen, insbesondere Fluor und Chlor, für Cyanomethyl, Cyanethyl, n- oder i-Cyanopropyl, für jeweils gegebenenfalls einfach bis dreifach, gleich oder verschieden durch Fluor, Chlor, Brom, Methyl und/oder Ethyl substituiertes Cyclopropyl, Cyclopropylmethyl, Cyclopentyl, Cyclopentylmethyl, Cyclohexyl oder Cyclohexylmethyl, oder für jeweils gegebenenfalls einfach bis dreifach, gleich oder verschieden durch Fluor, Chlor, Brom, Methyl, Ethyl und/oder Trifluormethyl substituiertes Phenyl, Benzyl, Phenylethyl, jeweils geradkettiges oder verzweigtes Phenylpropyl, Phenylbutyl, Phenylpentyl, Phenylhexyl oder für Morpholinyl-$C_1$-$C_4$-alkyl steht,

$R^2$ für Methyl, Ethyl, n-, i- oder Cyclopropyl, n-, i-, s-oder t-Butyl steht,

$R^3$ für Methyl, Ethyl, n- oder i-Propyl, n-, i-, s-oder t-Butyl, n- oder i-Pentyl steht,

X für Sauerstoff steht und

Y für Sauerstoff steht.

**4.** Verfahren zur Herstellung von substituierten 5-Alkoxy-1,2,4-triazol-3-(thi)onen der Formel (I) gemäß Anspruch 1,

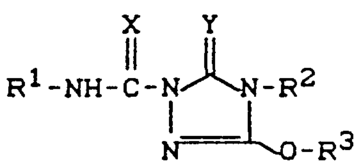

(I)

in welcher

$R^1$, $R^2$, $R^3$, X und Y die in Anspruch 1 genannten Bedeutungen haben,

dadurch gekennzeichnet, daß man

(a) 5-Alkoxy-1,2,4-triazol-3-(thi)one der allgemeinen Formel (II)

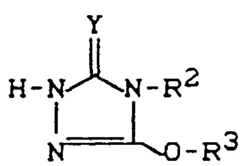

(II)

in welcher

$R^2$, $R^3$ und Y die oben angegebene Bedeutung haben,

mit Iso(thio)cyanaten der allgemeinen Formel (III)

$R^1$-N=C=X (III)

in welcher

$R^1$ und X die oben angegebene Bedeutung haben,

gegebenenfalls in Gegenwart eines Verdünnungsmittels und gegebenenfalls in Gegenwart eines basischen Reaktionshilfsmittels umsetzt, oder daß man

(b) 5-Alkoxyl-1,2,4-triazol-3-(thi)one der allgemeinen Formel (II)

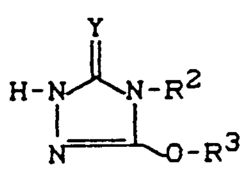

(II)

in welcher

$R^2$, $R^3$ und Y die oben angegebene Bedeutung haben,

mit reaktionsfähigen (Thio-)Carbamaten der allgemeinen Formel (IV)

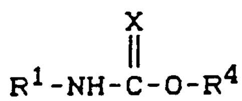 (IV)

in welcher

$R^1$ und X die oben angegebene Bedeutung haben und

$R^4$ für Alkyl, Aralkyl oder Aryl steht,

gegebenenfalls in Gegenwart eines Verdünnungsmittels und gegebenenfalls in Gegenwart eines basischen Reaktionshilfsmittesl umsetzt, oder daß man

(c) 5-Alkoxy-1,2,4-triazol-3-(thi)on-Derivate der allgemeinen Formel (V)

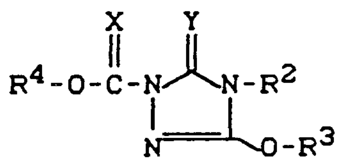 (V)

in welcher

$R^2$, $R^3$, X und Y die oben angegebene Bedeutung haben und

$R^4$ für Alkyl, Aralkyl oder Aryl steht,

mit Aminoverbindungen der allgemeinen Formel (VI)

$R^1$-$NH_2$ (VI)

in welcher

$R^1$ die oben angegebene Bedeutung hat,

gegebenenfalls in Gegenwart eines Verdünnungsmittels und gegebenenfalls in Gegenwart eines basischen Reaktionshilfsmittels umsetzt.

**5.** Herbizide Mittel, gekennzeichnet durch einen Gehalt an mindestens einem substituierten 5-Alkoxy-1,2,4-triazol-3-(thi)on-Derivat der Formel (I) gemäß den Ansprüchen 1 bis 4.

**6.** Verfahren zur Bekämpfung von unerwünschten Pflanzen, dadurch gekennzeichnet, daß man substituierte 5-Alkoxy-1,2,4-triazol-3-(thi)one der Formel (I) gemäß den Ansprüchen 1 bis 4 auf unerwünschte Pflanzen und/oder ihren Lebensraum einwirken läßt.

**7.** Verwendung von substituierten 5-Alkoxy-1,2,4-triazol-3-(thi)onen der Formel (I) gemäß den Ansprüchen 1 bis 4 zur Bekämpfung von unerwünschten Pflanzen.

**8.** Verfahren zur Herstellung von herbiziden Mitteln, dadurch gekennzeichnet, daß man substituierte 5-Alkoxy-1,2,4-triazol-3-(thi)one der Formel (I) gemäß den Ansprüchen 1 bis 4 mit Streckmitteln und/oder oberflächenaktiven Substanzen vermischt.

**9.** 5-Alkoxy-1,2,4-triazol-3-one der allgemeinen Formel (II) (Y = O)

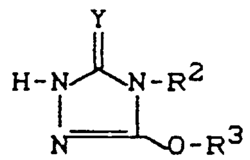

(II)

in welcher

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffatomen steht ausgenommen die Verbindung 5-Methoxy-4-methyl-2,4-dihydro-3H-1,2,4-triazol-3-on.

**10.** Verfahren zur Herstellung von 5-Alkoxy-1,2,4-triazol-3-onen der allgemeinen Formel (II) (Y = O)

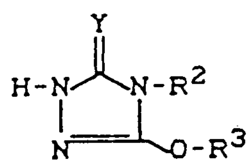

(II)

in welcher

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffstomen steht, ausgenommen die Verbindung 5-Methoxy-4-methyl-2,4-dihydro-3H-1,2,4-triazol-3-on,

dadurch gekennzeichnet, daß man die neuen 5-Alkoxy-1,2,4-triazol-3-one (II, Y = O) erhält, wenn man Hydrazino -ameisensäureester der allgemeinen Formel (VII)

$H_2N-NH-CO-O-R^4$ (VII)

in welcher

$R^4$ für Alkyl, Aralkyl oder Aryl steht,

mit Alkyliminokohlensäurediestern der allgemeinen Formel (VIII)

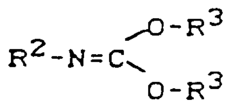

(VIII)

in welcher

$R^2$ und $R^3$ die oben angegebene Bedeutung haben,

gegebenenfalls in Gegenwart eines Verdünnungsmittels, bei Temperaturen zwischen 0 °C und 200 °C, umsetzt.

**Patentansprüche für folgenden Vertragsstaat : ES**

1. Verfahren zur Herstellung von substituierten 5-Alkoxy-1,2,4-triazol-3-(thi)onen der Formel (I) gemäß Anspruch 1,

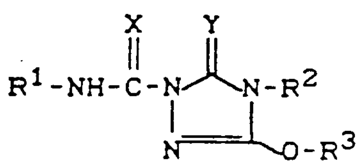

(I)

in welcher

$R^1$ für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten im cyclischen und gegebenenfalls im aliphatischen Teil ausgewählt sind: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem $R^1$ für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen -insbesondere Stickstoff, Sauerstoff und/oder Schwefel - im Heterocyclylteil steht, wobei als Substituenten ausgewählt sind: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem $R^1$ für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aryloxyalkyl, Arylalkenyl, Arylalkinyl, Aralkyloxy, Aryloxy, Aroyl oder Aryl mit jeweils 6 oder 10 Kohlenstoffatmen im

Arylteil und gegebenenfalls bis zu 8 Kohlenstoffatomen im Alkyl-, Alkenyl- oder Alkinylteil steht, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffatomen steht,

X für Sauerstoff oder Schwefel steht und

Y für Sauerstoff oder Schwefel steht

und Halogen in den in $R^1$, $R^2$ und $R^3$ genannten Substitutionsmustern für Fluor, Chlor, Brom und Iod steht,

dadurch gekennzeichnet, daß man

(a) 5-Alkoxy-1,2,4-triazol-3-(thi)one der allgemeinen Formel (II)

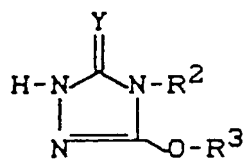

(II)

in welcher

$R^2$, $R^3$ und Y die oben angegebene Bedeutung haben,

mit Iso(thio)cyanaten der allgemeinen Formel (III)

$R^1$-N = C = X (III)

in welcher

$R^1$ und X die oben angegebene Bedeutung haben,

gegebenenfalls in Gegenwart eines Verdünnungsmittels und gegebenenfalls in Gegenwart eines basischen Reaktionshilfsmittels umsetzt, oder daß man

(b) 5-Alkoxy-1,2,4-triazol-3-(thi)one der allgemeinen Formel (II)

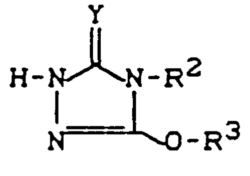

(II)

in welcher

$R^2$, $R^3$ und Y die oben angegebene Bedeutung haben,

mit reaktionsfähigen (Thio-)Carbamaten der allgemeinen Formel (IV)

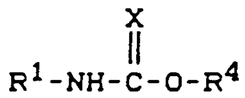

(IV)

in welcher

$R^1$ und X die oben angegebene Bedeutung haben und

$R^4$ für Alkyl, Aralkyl oder Aryl steht,

gegebenenfalls in Gegenwart eines Verdünnungsmittels und gegebenenfalls in Gegenwart eines basischen Reaktionshilfsmittesl umsetzt, oder daß man

(c) 5-Alkoxy-1,2,4-triazol-3-(thi)on-Derivate der allgemeinen Formel (V)

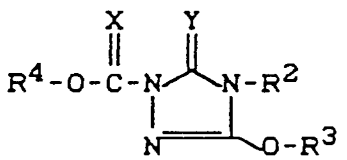

(V)

in welcher

$R^2$, $R^3$, X und Y die oben angegebene Bedeutung haben und

$R^4$ für Alkyl, Aralkyl oder Aryl steht,

mit Aminoverbindungen der allgemeinen Formel (VI)

$R^1$-$NH_2$ (VI)

in welcher

$R^1$ die oben angegebene Bedeutung hat,

gegebenenfalls in Gegenwart eines Verdünnungsmittels und gegebenenfalls in Gegenwart eines basischen Reaktionshilfsmittels umsetzt.

**2.** Verfahren zur Herstellung substituierter 5-Alkoxy-1,2,4-triazol-3-(thi)one der allgemeinen Formel (I) gemaß Anspruch 1, bei welchen

$R^1$ für Methyl, Ethyl, n- oder i-Propyl, n-, i-, s-oder t-Butyl, jeweils geradkettiges oder verzweigtes Pentyl, Hexyl, Heptyl, Octyl, Nonyl, Decyl oder Dodecyl, für Allyl, Propenyl, n- oder i-Butenyl, n- oder i-Pentenyl, n- oder i-Hexenyl, Propargyl, n-oder i-Butinyl, n-oder i-Pentinyl, n- oder i-Hexinyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 4 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclopentylmethyl, Cyclohexylmethyl, Cyclohexylethyl, Cyclohexenyl oder Cyclohexenylmethyl stehen, wobei als besonders bevorzugte Substituenten im cyclischen und gegebenenfalls im aliphatischen Teil ausgewählt sind: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl; außerdem $R^1$ für im Heterocyclylteil gegebenenfalls ein- bis dreifach, gleich oder verschieden substituiertes Heterocyclylmethyl, Heterocyclylethyl oder Heterocyclylpropyl steht, wobei als Heterocyclen jeweils in Frage kommen:

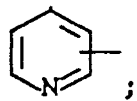 ; 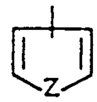 ; 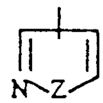 ; 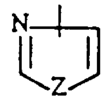 ;

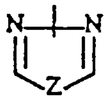 ; 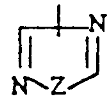 ; 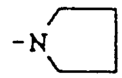 ; 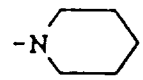 ;

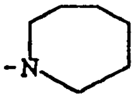 ; 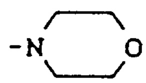 oder 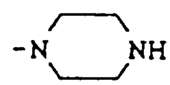

wobei Z jeweils für Sauerstoff oder Schwefel steht und wobei als besonders bevorzugte Substituenten ausgewählt sind:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

außerdem $R^1$ für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Phenoxymethyl, Phenoxyethyl, Phenoxypropyl, Phenoxybutyl, Phenylethinyl, Benzyloxy, Phenylethyloxy, Phenoxy, Benzoyl, Phenyl oder Naphthyl steht, wobei als Phenylsubstituenten jeweils in Frage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Difluormethoxy, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl und Phenoxy,

$R^2$ für Methyl, Ethyl, n- oder i-Propyl, n-, i-, s-oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl, Halogenalkenyl und Halogenalkinyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclopentylmethyl, Cyclohexylmethyl oder Cyclohexylethyl steht,

$R^3$ für Methyl, Ethyl, n- oder i-Propyl, n-, i-, s-oder t-Butyl, n-, i-, s- oder t-Pentyl, n- oder i-Hexyl, für Allyl, Propargyl, Methoxyethyl, Ethoxyethyl, Fluorethyl, Chlorethyl, Fluorpropyl, Chlorpropyl oder Cyanoethyl steht,

X für Sauerstoff oder Schwefel steht und

Y für Sauerstoff oder Schwefel steht.

**3.** Verfahren zur Herstellung substituierter 5-Alkoxy-1,2,4-triazol-3-(thi)one der allgemeinen Formel (I) gemäß Anspruch 1, bei welchen

$R^1$ für Methyl, Ethyl, n- oder i-Propyl, n-, i-, s-oder t-Butyl, n-, i-, s- oder t-Pentyl, n-, i-, s-oder t-Hexyl, Propargyl, n- oder i-Butinyl, n- oder i-Pentinyl, für geradkettiges oder verzweigtes Halogenalkyl mit 1 bis 4 Kohlenstoffatomen und 1 bis 4 gleichen oder verschiedenen Halogenatomen, insbesondere Fluor und Chlor, für Cyanomethyl, Cyanethyl, n- oder i-Cyanopropyl, für jeweils gegebenenfalls einfach bis dreifach, gleich oder verschieden durch Fluor, Chlor, Brom, Methyl und/oder Ethyl substituiertes Cyclopropyl, Cyclopropylmethyl, Cyclopentyl, Cyclopentylmethyl, Cyclohexyl oder Cyclohexylmethyl, oder für jeweils gegebenenfalls einfach bis dreifach, gleich oder verschieden durch Fluor, Chlor, Brom, Methyl, Ethyl und/oder Trifluormethyl substituiertes Phenyl, Benzyl, Phenylethyl, jeweils geradkettiges oder verzweigtes Phenylpropyl, Phenylbutyl, Phenylpentyl, Phenylhexyl oder für Morpholinyl-$C_1$-$C_4$-alkyl steht,

$R^2$ für Methyl, Ethyl, n-, i- oder Cyclopropyl, n-, i-, s-oder t-Butyl steht,

$R^3$ für Methyl, Ethyl, n- oder i-Propyl, n-, i-, s-oder t-Butyl, n- oder i-Pentyl steht,

X für Sauerstoff steht und
Y für Sauerstoff steht.

**4.** Herbizide Mittel, gekennzeichnet durch einen Gehalt an mindestens einem substituierten 5-Alkoxy-1,2,4-triazol-3-(thi)on-Derivat der Formel (I) gemäß den Ansprüchen 1 bis 3.

**5.** Verfahren zur Bekämpfung von unerwünschten Pflanzen, dadurch gekennzeichnet, daß man substituierte 5-Alkoxy-1,2,4-triazol-3-(thi)one der Formel (I) gemäß den Ansprüchen 1 bis 3 auf unerwünschte Pflanzen und/oder ihren Lebensraum einwirken läßt.

**6.** Verwendung von substituierten 5-Alkoxy-1,2,4-triazol-3-(thi)onen der Formel (I) gemäß den Ansprüchen 1 bis 3 zur Bekämpfung von unerwünschten Pflanzen.

**7.** Verfahren zur Herstellung von herbiziden Mitteln, dadurch gekennzeichnet, daß man substituierte 5-Alkoxy-1,2,4-triazol-3-(thi)one der Formel (I) gemäß den Ansprüchen 1 bis 3 mit Streckmitteln und/oder oberflächenaktiven Substanzen vermischt.

**8.** Verfahren zur Herstellung von 5-Alkoxy-1,2,4-triazol-3-onen der allgemeinen Formel (II) (Y = O)

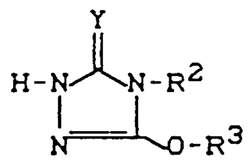

(II)

in welcher

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, Cyanoalkyl mit 1 bis 8 Kohlenstoffatomen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen oder für Cyanoalkyl mit 1 bis 8 Kohlenstoffatomen steht, ausgenommen die Verbindung 5-Methoxy-4-methyl-2,4-dihydro-3H-1,2,4-triazol-3-on, dadurch gekennzeichnet, daß man die neuen 5-Alkoxy-1,2,4-triazol-3-one (II, Y = O) erhält, wenn man Hydrazino -ameisensäureester der allgemeinen Formel (VII)

$H_2N\text{-}NH\text{-}CO\text{-}O\text{-}R^4$ (VII)

in welcher

$R^4$ für Alkyl, Aralkyl oder Aryl steht,

mit Alkyliminokohlensäurediestern der allgemeinen Formel (VIII)

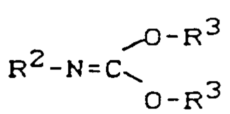

(VIII)

in welcher

$R^2$ und $R^3$ die oben angegebene Bedeutung haben,

gegebenenfalls in Gegenwart eines Verdünnungsmittels, bei Temperaturen zwischen 0 °C und 200 °C, umsetzt.

## Claims

### Claims for the following Contracting States : BE, CH, DE, DK, FR, GB, IT, LI, NL

**1.** Substituted 5-alkoxy-1,2,4-triazol-3(thi)ones of the general formula (I)

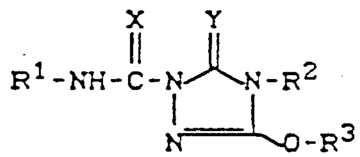

(I)

in which

$R^1$ represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoximinoalkyl, alkoxycarbonylalkyl or alkoxycarbonylalkenyl, each of which has up to 6 carbon atoms in the individual alkyl or alkenyl moieties, alkylaminoalkyl or dialkylaminoalkyl, each of which has 1 to 6 carbon atoms in the individual alkyl moieties, or represents cycloalkyl, cycloalkylalkyl, cycloalkenyl or cycloalkenylalkyl, each of which has 3 to 8 carbon atoms in the cycloalkyl or cycloalkenyl moiety and if appropriate 1 to 6 carbon atoms in the alkyl moiety and each of which is optionally monosubstituted or polysubstituted by identical or different substituents, the following being selected as substituents in the cyclic and optionally in the aliphatic moiety: halogen, cyano, and in each case straight-chain or branched alkyl or halogenoalkyl, each of which has 1 to 4 carbon atoms and if appropriate 1 to 9 identical or different halogen atoms, or in each case divalent alkanediyl or alkenediyl, each of which has up to 4 carbon atoms; $R^1$ furthermore represents heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and each of which is optionally monosubstituted or polysubstituted by identical or different substituents, the following being selected as substituents: halogen, cyano, nitro, and in each case straight-chain or branched alkyl, alkoxy, alkylthio, halogenoalkyl, halogenoalkoxy, halogenoalkylthio or alkoxycarbonyl, each of which has 1 to 5 carbon atoms and if appropriate 1 to 9 identical or different halogen atoms; $R^1$ furthermore represents in each case straight-chain or branched alkoxy having 1 to 8 carbon atoms, alkenyloxy having 2 to 8 carbon atoms or alkinyloxy having 2 to 8 carbon atoms, and finally represents aralkyl, aryloxyalkyl, arylalkenyl, arylalkinyl, aralkyloxy, aryloxy, aroyl or aryl, each of which has 6 or 10 carbon atoms in the aryl moiety and if appropriate up to 8 carbon atoms in the alkyl, alkenyl or alkinyl moiety, and each of which is optionally monosubstituted or polysubstituted by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms and if appropriate 1 to 9 identical or different halogen atoms, cycloalkyl having 3 to 6 carbon atoms and phenoxy, and suitable optional alkyl substituents being: halogen or cyano,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of

which has 1 to 6 carbon atoms in the individual alkyl moieties, cyanoalkyl having 1 to 8 carbon atoms, or represents cycloalkylalkyl or cycloalkyl, each of which has 3 to 7 carbon atoms in the cycloalkyl moiety and if appropriate 1 to 6 carbon atoms in the straight-chain or branched alkyl moiety,

$R^3$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cyanoalkyl having 1 to 8 carbon atoms,

X represents oxygen or sulphur and

Y represents oxygen or sulphur

and halogen in the substitution patterns mentioned in the case of $R^1$, $R^2$ and $R^3$ represents fluorine, chlorine, bromine and iodine.

**2.** Substituted 5-alkoxy-1,2,4-triazol-3-(thi)ones of the general formula (I) according to Claim 1, in which

$R^1$ represents methyl, ethyl, n- or i-propyl, n-, i-, s- or t-butyl, in each case straight-chain or branched pentyl, hexyl, heptyl, octyl, nonyl, decyl or dodecyl, or represents allyl, propenyl, n- or i-butenyl, n- or i-pentenyl, n- or i-hexenyl, propargyl, n- or i-butinyl, n- or i-pentinyl, n- or i-hexinyl, or represents straight-chain or branched halogenoalkyl having 1 to 4 carbon atoms and 1 to 9 identical or different halogen atoms, in particular fluorine, chlorine or bromine, or represents in each case straight-chain or branched halogenoalkenyl or halogenoalkinyl, each of which has 3 to 5 carbon atoms and 1 to 3 halogen atoms, in particular fluorine or chlorine, or represents in each case straight-chain or branched cyanoalkyl having 1 to 4 carbon atoms in the alkyl moiety, hydroxyalkyl having 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, each of which has up to 4 carbon atoms in the individual alkyl or alkenyl moieties, or represents cyclopropyl, cyclopropylmethyl, cyclopropylethyl, cyclobutyl, cyclopentyl, cyclohexyl, cycloheptyl, cyclooctyl, cyclopentylmethyl, cyclohexylmethyl, cyclohexylethyl, cyclohexenyl or cyclohexenylmethyl, each of which is optionally monosubstituted to trisubstituted by identical or different substituents, the following being selected as particularly preferred substituents in the cyclic and if appropriate in the aliphatic moiety: fluorine, chlorine, bromine, methyl, ethyl, n- or i-propyl, n-, i-, s- or t-butyl, cyano, methanediyl, ethanediyl, butanediyl or butadienediyl; $R^1$ furthermore represents heterocyclylmethyl, heterocyclylethyl or heterocyclylpropyl, each of which is optionally monosubstituted to trisubstituted in the heterocyclyl moiety by identical or different substituents, suitable heterocycles in each case being:

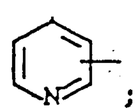 ; 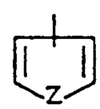 ; 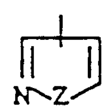 ; 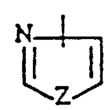 ;

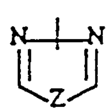 ; 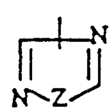 ; 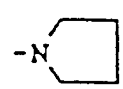 ; 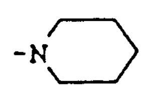 ;

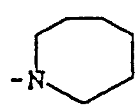 ; 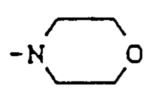 or 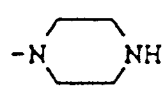

where Z in each case represents oxygen or sulphur and where the following are selected as particularly preferred substituents:

fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^1$ furthermore represents in each case straight-chain or branched alkoxy having 1 to 6 carbon atoms, alkenyloxy having 3 to 6 carbon atoms or alkinyloxy having 3 to 6 carbon atoms, or represents benzyl, phenylethyl, phenylpropyl, phenylbutyl, phenylpentyl, phenylhexyl, phenylheptyl, phenylcyanomethyl, phenylcyanoethyl, phenylcyanopropyl, phenoxymethyl, phenoxyethyl, phenoxypropyl, phenoxybutyl, phenylethinyl, benzyloxy, phenylethyloxy, phenoxy, benzoyl, phenyl or naphthyl, if appropriate straight-chain or branched, each of which is optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, difluoromethoxy, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl and phenoxy,

$R^2$ represents methyl, ethyl, n- or i-propyl, n-, i-, s- or t-butyl, n- or i-pentyl, n- or i-hexyl, or represents allyl, propargyl, methoxy, ethoxy, methoxymethyl, ethoxymethyl, methoxyethyl or ethoxyethyl, or represents straight-chain or branched halogenoalkyl, halogenoalkenyl and halogenoalkinyl having 1 to 4 carbon atoms and 1 to 9 identical or different halogen atoms, in particular fluorine, chlorine or bromine, or represents cyclopentyl, cyclohexyl, cyclopropyl, cyclopropylmethyl, cyclopentylmethyl, cyclohexylmethyl or cyclohexylethyl,

$R^3$ represents methyl, ethyl, n- or i-propyl, n-, i-, s- or t-butyl, n-, i-, s-or t-pentyl, n- or i-hexyl, or represents allyl, propargyl, methoxyethyl, ethoxyethyl, fluoroethyl, chloroethyl, fluoropropyl, chloropropyl or cyanoethyl,

X represents oxygen or sulphur and

Y represents oxygen or sulphur.

**3.** Substituted 5-alkoxy-1,2,4-triazol-3-(thi)ones of the general formula (I) according to Claim 1, in which

$R^1$ represents methyl, ethyl, n- or i-propyl, n-, i-, s- or t-butyl, n-, i-, s- or t-pentyl, n-, i-, s- or t-hexyl, propargyl, n- or i-butinyl, n- or i-pentinyl, or represents straight-chain or branched halogenoalkyl having 1 to 4 carbon atoms and 1 to 4 identical or different halogen atoms, in particular fluorine and chlorine, or represents cyanomethyl, cyanoethyl or n- or i-cyanopropyl, or represents cyclopropyl, cyclopropylmethyl, cyclopentyl, cyclopentylmethyl, cyclohexyl or cyclohexylmethyl, each of which is optionally monosubstituted to trisubstituted by identical or different substituents, or represents phenyl, benzyl or phenylethyl, each of which is optionally monosubstituted to trisubstituted by identical or different substituents from the series comprising fluorine, chlorine, bromine, methyl, ethyl and/or trifluoromethyl, or represents in each case straight-chain or branched phenylpropyl, phenylbutyl, phenylpentyl or phenylhexyl, or represents morpholinyl-$C_1$-$C_4$-alkyl,

$R^2$ represents methyl, ethyl, n-, i- or cyclopropyl, n-, i-, s- or t-butyl,

$R^3$ represents methyl, ethyl, n- or i-propyl, n-, i-, s- or t-butyl, n- or i-pentyl,

X represents oxygen and

Y represents oxygen.

**4.** Process for the preparation of substituted 5-alkoxy-1,2,4-triazol-3-(thi)ones of the formula (I) according to Claim 1,

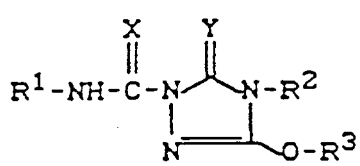

(I)

in which

$R^1$, $R^2$, $R^3$, X and Y have the meanings mentioned in Claim 1,

characterized in that

a) 5-alkoxy-1,2,4-triazol-3-(thi)ones of the general formula (II)

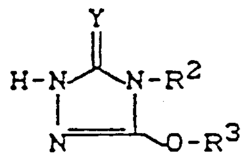

(II)

in which

$R^2$, $R^3$ and Y have the abovementioned meaning,

are reacted with iso(thio)cyanates of the general formula (III)

$R^1$-N=C=X (III)

in which

$R^1$ and X have the abovementioned meaning,

if appropriate in the presence of a diluent and if appropriate in the presence of a basic reaction auxiliary, or in that

(b) 5-alkoxy-1,2,4-triazol-3(thi)ones of the general formula (II)

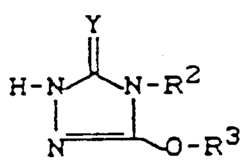

(II)

in which

$R^2$, $R^3$ and Y have the abovementioned meaning

are reacted with reactive (thio-)carbamates of the general formula (IV)

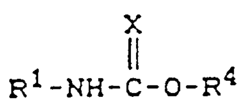

(IV)

in which

$R^1$ and X have the abovementioned meaning and

$R^4$ represents alkyl, aralkyl or aryl,

if appropriate in the presence of a diluent and if appropriate in the presence of a basic reaction auxiliary, or in that

(c) 5-alkoxy-1,2,4-triazol-3-(thi)one derivatives of the general formula (V)

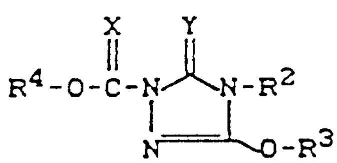

(V)

in which

$R^2$, $R^3$, X and Y have the abovementioned meaning and

$R^4$ represents alkyl, aralkyl or aryl,

are reacted with amino compounds of the general formula (VI)

$R^1$-$NH_2$ (VI)

in which

$R^1$ has the abovementioned meaning,

if appropriate in the presence of a diluent and if appropriate in the presence of a basic reaction auxiliary.

**5.** Herbicidal agents, characterized in that they contain at least one substituted 5-alkoxy-1,2,4-triazol-3-(thi)one derivative of the formula (I) according to Claims 1 to 4.

**6.** Method of combating undesired plants, characterized in that substituted 5-alkoxy-1,2,4-triazol-3-(thi)-ones of the formula (I) according to Claims 1 to 4 are allowed to act on undesired plants and/or their environment.

**7.** Use of substituted 5-alkoxy-1,2,4-triazol-3-(thi)ones of the formula (I) according to Claims 1 to 4 for combating undesired plants.

**8.** Process for the preparation of herbicidal agents, characterized in that substituted 5-alkoxy-1,2,4-triazol-3-(thi)ones of the formula (I) according to Claims 1 to 4 are mixed with extenders and/or surface-active substances.

**9.** 5-Alkoxy-1,2,4-triazol-3-ones of the general formula (II) (Y = O)

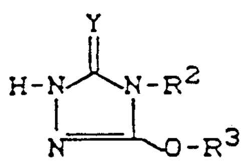

(II)

in which

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, cyanoalkyl having 1 to 8 carbon atoms, or represents cycloalkylalkyl or cycloalkyl, each of which has 3 to 7 carbon atoms in the cycloalkyl moiety and if appropriate 1 to 6 carbon atoms in the straight-chain or branched alkyl moiety,

$R^3$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cyanoalkyl having 1 to 8 carbon atoms, except for the compound 5-methoxy-4-methyl-2,4-dihydro-3H-1,2,4-triazol-3-one.

**10.** Process for the preparation of 5-alkoxy-1,2,4-triazol-3-ones of the general formula (II) (Y = O)

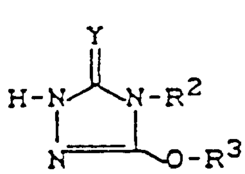

(II)

in which

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, cyanoalkyl having 1 to 8 carbon atoms, or represents cycloalkylalkyl or cycloalkyl, each of which has 3 to 7 carbon atoms in the cycloalkyl moiety and if appropriate 1 to 6 carbon atoms in the straight-chain or branched alkyl moiety,

$R^3$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cyanoalkyl having 1 to 8 carbon atoms, except for the compound 5-methoxy-4-methyl-2,4-dihydro-3H-1,2,4-triazol-3-one,

characterized in that the new 5-alkoxy-1,2,4-triazol-3-ones (II, Y = O) are obtained when hydrazinoformic esters of the general formula (VII)

$H_2N\text{-}NH\text{-}CO\text{-}O\text{-}R^4$ (VII)

in which

$R^4$ represents alkyl, aralkyl or aryl,

are reacted with alkyliminocarbonic diesters of the general formula (VIII)

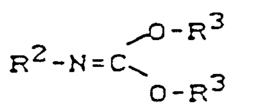

(VIII)

in which

$R^2$ and $R^3$ have the abovementioned meaning,

at temperatures between 0 °C and 200 °C, if appropriate in the presence of a diluent.

**Claims for the following Contracting State : ES**

**1.** Process for the preparation of substituted 5-alkoxy-1,2,4-triazol-3-(thi)ones of the formula (I) according to Claim 1,

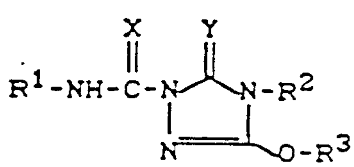

(I)

in which

$R^1$ represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoximinoalkyl, alkoxycarbonylalkyl or alkoxycarbonylalkenyl, each of which has up to 6 carbon atoms in the individual alkyl or alkenyl moieties, alkylaminoalkyl or dialkylaminoalkyl, each of which has 1 to 6 carbon atoms in the individual alkyl moieties, or represents cycloalkyl, cycloalkylalkyl, cycloalkenyl or cycloalkenylalkyl,

each of which has 3 to 8 carbon atoms in the cycloalkyl or cycloalkenyl moiety and if appropriate 1 to 6 carbon atoms in the alkyl moiety and each of which is optionally monosubstituted or polysubstituted by identical or different substituents, the following being selected as substituents in the cyclic and optionally in the aliphatic moiety: halogen, cyano, and in each case straight-chain or branched alkyl or halogenoalkyl, each of which has 1 to 4 carbon atoms and if appropriate 1 to 9 identical or different halogen atoms, or in each case divalent alkanediyl or alkenediyl, each of which has up to 4 carbon atoms; $R^1$ furthermore represents heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and each of which is optionally monosubstituted or polysubstituted by identical or different substituents, the following being selected as substituents: halogen, cyano, nitro, and in each case straight-chain or branched alkyl, alkoxy, alkylthio, halogenoalkyl, halogenoalkoxy, halogenoalkylthio or alkoxycarbonyl, each of which has 1 to 5 carbon atoms and if appropriate 1 to 9 identical or different halogen atoms; $R^1$ furthermore represents in each case straight-chain or branched alkoxy having 1 to 8 carbon atoms, alkenyloxy having 2 to 8 carbon atoms or alkinyloxy having 2 to 8 carbon atoms, and finally represents aralkyl, aryloxyalkyl, arylalkenyl, arylalkinyl, aralkyloxy, aryloxy, aroyl or aryl, each of which has 6 or 10 carbon atoms in the aryl moiety and if appropriate up to 8 carbon atoms in the alkyl, alkenyl or alkinyl moiety, and each of which is optionally monosubstituted or polysubstituted by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 2carbon atoms and if appropriate 1 to 9 identical or different halogen atoms, cycloalkyl having 3 to 6 carbon atoms and phenoxy, and suitable optional alkyl substituents being: halogen or cyano,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, cyanoalkyl having 1 to 8 carbon atoms, or represents cycloalkylalkyl or cycloalkyl, each of which has 3 to 7 carbon atoms in the cycloalkyl moiety and if appropriate 1 to 6 carbon atoms in the straight-chain or branched alkyl moiety,

$R^3$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cyanoalkyl having 1 to 8 carbon atoms,

X represents oxygen or sulphur and

Y represents oxygen or sulphur

and halogen in the substitution patterns mentioned in the case of $R^1$, $R^2$ and $R^3$ represents fluorine, chlorine, bromine and iodine,

characterized in that

a) 5-alkoxy-1,2,4-triazol-3-(thi)ones of the general formula (II)

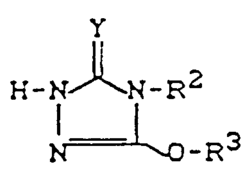 (II)

in which

$R^2$, $R^3$ and Y have the abovementioned meaning,

are reacted with iso(thio)cyanates of the general formula (III)

$R^1$-N=C=X (III)

in which

$R^1$ and X have the abovementioned meaning,

if appropriate in the presence of a diluent and if appropriate in the presence of a basic reaction auxiliary, or in that

(b) 5-alkoxy-1,2,4-triazol-3-(thi)ones of the general formula (II)

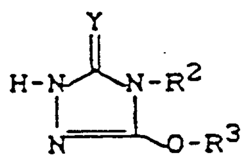

(II)

in which

$R^2$, $R^3$ and Y have the abovementioned meaning

are reacted with reactive (thio-)carbamates of the general formula (IV)

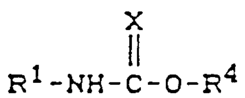

(IV)

in which

$R^1$ and X have the abovementioned meaning and

$R^4$ represents alkyl, aralkyl or aryl,

if appropriate in the presence of a diluent and if appropriate in the presence of a basic reaction auxiliary, or in that

(c) 5-alkoxy-1,2,4-triazol-3-(thi)one derivatives of the general formula (V)

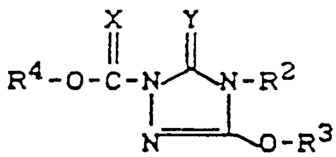

(V)

in which

$R^2$, $R^3$, X and Y have the abovementioned meaning and

$R^4$ represents alkyl, aralkyl or aryl,

are reacted with amino compounds of the general formula (VI)

$R^1$-$NH_2$ (VI)

in which

$R^1$ has the abovementioned meaning,

if appropriate in the presence of a diluent and if appropriate in the presence of a basic reaction auxiliary.

2. Process for the preparation of substituted 5-alkoxy-1,2,4-triazol-3-(thi)ones of the general formula (I) according to Claim 1, in which

$R^1$ represents methyl, ethyl, n- or i-propyl, n-, i-, s- or t-butyl, in each case straight-chain or branched pentyl, hexyl, heptyl, octyl, nonyl, decyl or dodecyl, or represents allyl, propenyl, n- or i-butenyl, n- or i-pentenyl, n- or i-hexenyl, propargyl, n- or i-butinyl, n- or i-pentinyl, n- or i-hexinyl, or represents straight-chain or branched halogenoalkyl having 1 to 4 carbon atoms and 1 to 9 identical or different halogen atoms, in particular fluorine, chlorine or bromine, or represents in each case straight-chain or branched halogenoalkenyl or halogenoalkinyl, each of which has 3 to 5 carbon atoms and 1 to 3 halogen atoms, in particular fluorine or chlorine, or represents in each case straight-chain or branched cyanoalkyl having 1 to 4 carbon atoms in the alkyl moiety, hydroxyalkyl having 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, each of which has up to 4 carbon atoms in the individual alkyl or alkenyl moieties, or represents cyclopropyl, cyclopropylmethyl, cyclopropylethyl, cyclobutyl, cyclopentyl, cyclohexyl, cycloheptyl, cyclooctyl, cyclopentylmethyl, cyclohexylmethyl, cyclohexylethyl, cyclohexenyl or cyclohexenylmethyl, each of which is optionally monosubstituted to trisubstituted by identical or different substituents, the following being selected as particularly preferred substituents in the cyclic and if appropriate in the aliphatic moiety: fluorine, chlorine, bromine, methyl, ethyl, n- or i-propyl, n-, i-, s- or t-butyl, cyano, methanediyl, ethanediyl, butanediyl or butadienediyl; $R^1$ furthermore represents heterocyclylmethyl, heterocyclylethyl or heterocyclylpropyl, each of which is optionally monosubstituted to trisubstituted in the heterocyclyl moiety by identical or different substituents, suitable heterocycles in each case being:

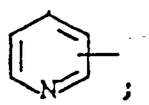 ; 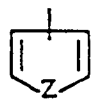 ; 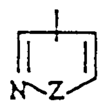 ; 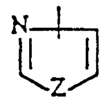 ;

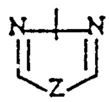 ; 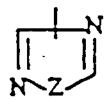 ; 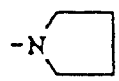 ; 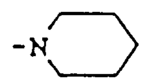 ;

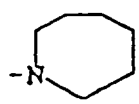 ; 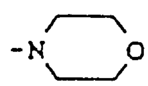 or 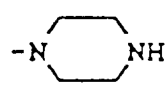

where Z in each case represents oxygen or sulphur and where the following are selected as particularly preferred substituents: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^1$ furthermore represents in each case straight-chain or branched alkoxy having 1 to 6 carbon atoms, alkenyloxy having 3 to 6 carbon atoms or alkinyloxy having 3 to 6 carbon atoms, or represents benzyl, phenylethyl, phenylpropyl, phenylbutyl, phenylpentyl, phenylhexyl, phenylheptyl, phenylcyanomethyl, phenylcyanoethyl, phenylcyanopropyl, phenoxymethyl, phenoxyethyl, phenoxypropyl, phenoxybutyl, phenylethinyl, benzyloxy, phenylethyloxy, phenoxy, benzoyl, phenyl or naphthyl, if appropriate straight-chain or branched, each of which is optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, difluoromethoxy, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl and phenoxy,

$R^2$ represents methyl, ethyl, n- or i-propyl, n-, i-, s- or t-butyl, n- or i-pentyl, n- or i-hexyl, or represents allyl, propargyl, methoxy, ethoxy, methoxymethyl, ethoxymethyl, methoxyethyl or ethoxyethyl, or represents straight-chain or branched halogenoalkyl, halogenoalkenyl and halogenoalkinyl having 1 to 4 carbon atoms and 1 to 9 identical or different halogen atoms, in particular fluorine, chlorine or bromine, or represents cyclopentyl, cyclohexyl, cyclopropyl, cyclopropylmethyl, cyclopentylmethyl, cyclohexylmethyl or cyclohexylethyl,

$R^3$ represents methyl, ethyl, n- or i-propyl, n-, i-, s- or t-butyl, n-, i-, s-or t-pentyl, n- or i-hexyl, or represents allyl, propargyl, methoxyethyl, ethoxyethyl, fluoroethyl, chloroethyl, fluoropropyl,

chloropropyl or cyanoethyl,
X represents oxygen or sulphur and
Y represents oxygen or sulphur.

**3.** Process for the preparation of substituted 5-alkoxy-1,2,4-triazol-3-(thi)ones of the general formula (I) according to Claim 1, in which
$R^1$ represents methyl, ethyl, n- or i-propyl, n-, i-, s- or t-butyl, n-, i-, s- or t-pentyl, n-, i-, s- or t-hexyl, propargyl, n- or i-butinyl, n- or i-pentinyl, or represents straight-chain or branched halogenoalkyl having 1 to 4 carbon atoms and 1 to 4 identical or different halogen atoms, in particular fluorine and chlorine, or represents cyanomethyl, cyanoethyl or n- or i-cyanopropyl, or represents cyclopropyl, cyclopropylmethyl, cyclopentyl, cyclopentylmethyl, cyclohexyl or cyclohexylmethyl, each of which is optionally monosubstituted to trisubstituted by identical or different substituents, or represents phenyl, benzyl or phenylethyl, each of which is optionally monosubstituted to trisubstituted by identical or different substituents from the series comprising fluorine, chlorine, bromine, methyl, ethyl and/or trifluoromethyl, or represents in each case straight-chain or branched phenylpropyl, phenylbutyl, phenylpentyl or phenylhexyl, or represents morpholinyl-$C_1$-$C_4$-alkyl,
$R^2$ represents methyl, ethyl, n-, i- or cyclopropyl, n-, i-, s- or t-butyl,
$R^3$ represents methyl, ethyl, n- or i-propyl, n-, i-, s- or t-butyl, n- or i-pentyl,
X represents oxygen and
Y represents oxygen.

**4.** Herbicidal agents, characterized in that they contain at least one substituted 5-alkoxy-1,2,4-triazol-3-(thi)one derivative of the formula (I) according to Claims 1 to 3.

**5.** Method of combating undesired plants, characterized in that substituted 5-alkoxy-1,2,4-triazol-3-(thi)-ones of the formula (I) according to Claims 1 to 3 are allowed to act on undesired plants and/or their environment.

**6.** Use of substituted 5-alkoxy-1,2,4-triazol-3-(thi)ones of the formula (I) according to Claims 1 to 3 for combating undesired plants.

**7.** Process for the preparation of herbicidal agents, characterized in that substituted 5-alkoxy-1,2,4-triazol-3-(thi)ones of the formula (I) according to Claims 1 to 3 are mixed with extenders and/or surface-active substances.

**8.** Process for the preparation of 5-alkoxy-1,2,4-triazol-3-ones of the general formula (II) (Y = O)

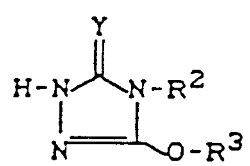

(II)

in which
$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, cyanoalkyl having 1 to 8 carbon atoms, or represents cycloalkylalkyl or cycloalkyl, each of which has 3 to 7 carbon atoms in the cycloalkyl moiety and if appropriate 1 to 6 carbon atoms in the straight-chain or branched alkyl moiety,
$R^3$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8

carbon atoms and 1 to 15 identical or different halogen atoms, halogenoalkinyl having 2 to 8 carbon atoms and 1 to 13 identical or different halogen atoms, alkoxyalkyl having 1 to 6 carbon atoms in each of the individual alkyl moieties, or represents cyanoalkyl having 1 to 8 carbon atoms, except for the compound 5-methoxy-4-methyl-2,4-dihydro-3H-1,2,4-triazol-3-one,

characterized in that the new 5-alkoxy-1,2,4-triazol-3-ones (II, Y = O) are obtained when hydrazinoformic esters of the general formula (VII)

$H_2N-NH-CO-O-R^4$ (VII)

in which

$R^4$ represents alkyl, aralkyl or aryl,

are reacted with alkyliminocarbonic diesters of the general formula (VIII)

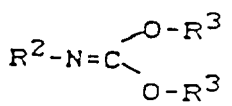

(VIII)

in which

$R^2$ and $R^3$ have the above mentioned meaning,

at temperatures between 0 °C and 200 °C, if appropriate in the presence of a diluent.

## Revendications

## Revendications pour les Etats contractants suivants : BE, CH, DE, DK, FR, GB, IT, LI, NL

**1.** 5-alkoxy-1,2,4-triazole-3-(thi)ones substituées de formule générale (I)

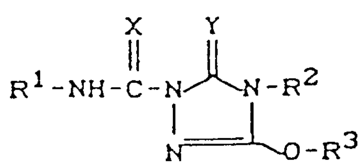

(I)

dans laquelle

$R^1$ est un groupe, linéaire ou ramifié, alkyle ayant 1 à 18 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ou halogénalcynyle ayant chacun 2 à 8 atomes de carbone et 1 à 15 et respectivement 13 atomes d'halogènes identiques ou différents, cyanalkyle ayant 1 à 8 atomes de carbone, hydroxyalkyle ayant 1 à 8 atomes de carbone et 1 à 6 groupes hydroxy, alkoxyalkyle, alkoxyminoalkyle, alkoxycarbonylalkyle ou alkoxycarbonylalcényle ayant chacun jusqu'à 6 atomes de carbone dans les parties alkyle et alcényle individuelles, alkylaminoalkyle ou dialkylaminoalkyle ayant chacun 1 à 6 atomes de carbone dans les parties alkyle individuelles ou un groupe cycloalkyle, cycloalkylalkyle, cycloalcényle ou cycloalcénylalkyle ayant chacun 3 à 8 atomes de carbone dans la partie cycloalkyle ou cycloalcényle et le cas échéant 1 à 6 atomes de carbone dans la partie alkyle et portant chacun le cas échéant un ou plusieurs substituants identiques ou différents, en choisissant comme substituants dans la partie cyclique et le cas échéant dans la partie aliphatique : un halogène, un radical cyano ainsi qu'un radical alkyle ou halogénalkyle linéaire ou ramifié ayant chacun 1 à 4 atomes de carbone et le cas échéant 1 à 9 atomes d'halogènes identiques ou différents ou un radical alcanediyle ou alcènediyle divalent ayant chacun jusqu'à 4 atomes de carbone ; en outre, $R^1$ représente un groupe hétérocyclylalkyle portant le cas échéant un ou plusieurs substituants identiques ou différents dans la partie hétérocyclyle et ayant 1 à 6 atomes de carbone dans la partie alkyle linéaire ou ramifiée et 1 à 9 atomes de carbone ainsi que 1 à 3 hétéroatomes - notamment d'azote, d'oxygène et/ou de soufre - dans la partie hétérocyclyle, en choisissant

comme substituants : un halogène, un radical cyano, nitro ainsi qu'un radical alkyle, alkoxy, alkylthio, halogénalkyle, halogénalkoxy, halogénalkylthio ou alkoxycarbonyle linéaire ou ramifié ayant chacun 1 à 5 atomes de carbone et le cas échéant 1 à 9 atomes d'halogènes identiques ou différents ; en outre, $R^1$ représente un groupe, linéaire ou ramifié, alkoxy ayant 1 à 8 atomes de carbone, alcényloxy ayant 2 à 8 atomes de carbone ou alcynyloxy ayant 2 à 8 atomes de carbone et enfin un groupe aralkyle, aryloxyalkyle, arylacényle, arylalcynyle, aralkyloxy, aryloxy, aroyle ou aryle ayant chacun 6 à 10 atomes de carbone dans la partie aryle et le cas échéant jusqu'à 8 atomes de carbone dans la partie alkyle, alcényle ou alcynyle et portant chacun le cas échéant un ou plusieurs substituants identiques ou différents, en considérant comme substituants de la partie aryle : un halogène, un radical cyano, nitro, hydroxy, un radical alkyle, alkoxy, alkylthio, halogénalkyle, halogénalkoxy, halogénalkylthio, alkylsulfinyle, alkylsulfonyle, halogénalkylsulfinyle, halogénalkylsulfonyle, alcanoyle ou alkoxycarbonyle linéaire ou ramifié ayant chacun 1 à 6 atomes de carbone et le cas échéant 1 à 9 atomes d'halogènes identiques ou différents, un radical cycloalkyle ayant 3 à 6 atomes de carbone et un radical phénoxy, et en considérant le cas échéant comme substituants de la partie alkyle : un halogène ou un radical cyano,

$R^2$ représente un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, cyanalkyle ayant 1 à 8 atomes de carbone, un groupe cycloalkylalkyle ou cycloalkyle ayant chacun 3 à 7 atomes de carbone dans la partie cycloalkyle et le cas échéant 1 à 6 atomes de carbone dans la partie alkyle linéaire ou ramifiée,

$R^3$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ayant 1 à 6 atomes de carbone dans chacune des parties alkyle individuelles ou cyanalkyle ayant 1 à 8 atomes de carbone,

X représente de l'oxygène ou du soufre et

Y est de l'oxygène ou du soufre et l'halogène dans les modèles de substitution mentionnés dans $R^1$, $R^2$ et $R^3$ représente du fluor, du chlore, du brome et de l'iode.

**2.** 5-alkoxy-1,2,4-triazole-3-(thi)ones subsituées de formule générale (I) suivant la revendication 1, dans lesquelles

$R^1$ représente un groupe méthyle, éthyle, n-propyle, isopropyle, n-butyle, isobutyle, sec.-butyle, tertio-butyle, un groupe pentyle, hexyle, heptyle, octyle, nonyle, décyle ou dodécyle linéaire ou ramifié, un groupe allyle, propényle, n-butényle, isobutényle, n-pentényle, isopentényle, n-hexényle, isohexényle, propargyle, n-butynyle, isobutynyle, n-pentynyle, isopentynyle, n-hexynyle, ou isohexynyle, un groupe halogénalkyle linéaire ou ramifié ayant 1 à 4 atomes de carbone et 1 à 9 atomes d'halogènes identiques ou différents, notamment fluor, chlore ou brome, un groupe halogénalcényle ou halogénalcynyle linéaire ou ramifié ayant chacun 3 à 5 atomes de carbone et 1 à 3 atomes d'halogènes, notamment fluor ou chlore, un groupe, linéaire ou ramifié, cyanalkyle ayant 1 à 4 atomes de carbone dans la partie alkyle, hydroxyalkyle ayant 1 à 6 atomes de carbone et 1 à 3 groupes hydroxy, alkoxyalkyle, alkoxyminoalkyle, alkoxycarbonylalkyle ou alkoxycarbonylalcényle, alkylaminoalkyle ou dialkylaminoalkyle ayant chacun jusqu'à 4 atomes de carbone dans les parties alkyle et alcényle individuelles, ou un groupe cyclopropyle, cyclopropylméthyle, cyclopropyléthyle, cyclobutyle, cyclopentyle, cyclohexyle, cycloheptyle, cyclooctyle, cyclopentylméthyle, cyclohexylméthyle, cyclohexyléthyle, cyclohexényle ou cyclohexénylméthyle portant chacun le cas échéant 1 à 3 substituants identiques ou différents, en choisissant comme substituants particulièrement appréciés dans la qartie cyclique et le cas échéant dans la partie aliphatique : le fluor, le chlore, le brome, un radical méthyle, éthyle, n-propyle, isopropyle, n-butyle, isobutyle, sec.-butyle, tertio-butyle, cyano, méthanediyle, éthanediyle, butanediyle ou butadiènediyle ; en

outre, $R^1$ représente un groupe hétérocyclylméthyle, hétérocyclyléthyle ou hétérocyclylpropyle portant le cas échéant dans la partie hétérocyclyle 1 à 3 substituants identiques ou différents, en considérant dans chaque cas comme hétérocycliques :

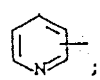 ; 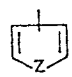 ; 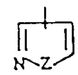 ; 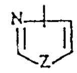 ;

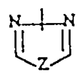 ; 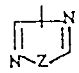 ; 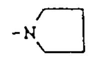 ; 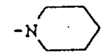 ;

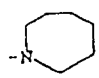 ; 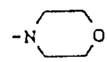 ou 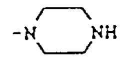

où Z représente dans chaque cas de l'oxygène ou du soufre et en choisissant comme substituants particulièrement appréciés : le fluor, le chlore, le brome, un radical cyano, nitro, méthyle, éthyle, n-propyle, isopropyle, n-butyle, isobutyle, sec.-butyle, tertio-butyle, méthoxy, éthoxy, méthylthio, trifluorométhyle, trifluorométhoxy ou trifluorométhylthio ; en outre, $R^1$ représente un groupe, linéaire ou ramifié, alkoxy ayant 1 à 6 atomes de carbone, alcényloxy ayant 3 à 6 atomes de carbone ou alcynyloxy ayant 3 à 6 atomes de carbone ou un groupe benzyle, phényléthyle, phénylpropyle, phénylbutyle, phénylpentyle, phénylhexyle, phénylheptyle, phénylcyanométhyle, phénylcyanéthyle, phénylcyanopropyle, phénoxyméthyle, phénoxyéthyle, phénoxypropyle, phénoxybutyle, phényléthynyle, benzyloxy, phényléthyloxy, phénoxy, benzoyle, phényle ou naphtyle, le cas échéant linéaire ou ramifié et portant chacun éventuellement 1 à 3 substituants identiques ou différents, en considérant comme substituants de la partie phényle : le fluor, le chlore, le brome, un radical hydroxy, cyano, nitro, méthyle, éthyle, n-propyle, isopropyle, n-butyle, isobutyle, sec.-butyle, tertio-butyle, méthoxy, éthoxy, méthylthio, trifluorométhyle, difluorométhoxy, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle et phénoxy,

$R^2$ est un groupe méthyle, éthyle, n-propyle, isopropyle, n-butyle, isobutyle, sec.-butyle, tertio-butyle, n-pentyle, isopentyle, n-hexyle ou isohexyle, un groupe allyle, propargyle, méthoxy, éthoxy, méthoxyméthyle, éthoxyméthyle, méthoxyéthyle, éthoxyéthyle, un groupe halogénalkyle, halogénalcényle ou halogénalcynyle linéaire ou ramifié ayant chacun 1 à 4 atomes de carbone et 1 à 9 atomes d'halogènes identiques ou différents, notamment fluor, chlore ou brome, un groupe cyclopentyle, cyclohexyle, cyclopropyle, cyclopropylméthyle, cyclopentylméthyle, cyclohexylméthyle ou cyclohexyléthyle,

$R^3$ est un groupe méthyle, éthyle, n-propyle, isopropyle, n-butyle, isobutyle, sec.-butyle, tertio-butyle, n-pentyle, isopentyle, sec.-pentyle, tertio-pentyle, n-hexyle ou isohexyle, un groupe allyle, propargyle, méthoxyéthyle, éthoxyéthyle, fluoréthyle, chloréthyle, fluoropropyle, chloropropyle ou cyanéthyle,

X est de l'oxygène ou du soufre et

Y est de l'oxygène ou du soufre.

**3.** 5-alkoxy-1,2,4-triazole-3-(thi)ones substituées de formule générale (I) suivant la revendication 1, dans lesquelles

$R^1$ est un groupe méthyle, éthyle, n-propyle, isopropyle, n-butyle, isobutyle, sec.-butyle, tertio-butyle, n-pentyle, isopentyle, sec.-pentyle, tertio-pentyle, n-hexyle, isohexyle, sec.-hexyle, tertio-hexyle, propargyle, n-butynyle, isobutynyle, n-pentynyle ou isopentynyle, un groupe halogénalkyle linéaire ou ramifié ayant 1 à 4 atomes de carbone et 1 à 4 atomes d'halogènes identiques ou différents, notamment fluor et chlore, un groupe cyanométhyle, cyanéthyle, n-cyanopropyle ou isocyanopropyle, un groupe cyclopropyle, cyclopropylméthyle, cyclopentyle, cyclopentylméthyle, cyclohexyle ou cyclohexylméthyle portant chacun le cas échéant 1 à 3 substituants fluoro, chloro, bromo, méthyle et/ou éthyle identiques ou différents ou un groupe phényle, benzyle, phényléthyle portant chacun le cas échéant 1 à 3 substituants fluoro, chloro, bromo, méthyle, éthyle et/ou trifluorométhyle identiques ou différents, un groupe

phénylpropyle, phénylbutyle, phénylpentyle, phénylhexyle, ou morpholinyl-(alkyle en $C_1$ à $C_4$) linéaire ou ramifié,

$R^2$ est un groupe méthyle, éthyle, n-propyle, isopropyle, cyclopropyle, n-butyle, isobutyle, sec.-butyle ou tertio-butyle,

$R^3$ est un groupe méthyle, éthyle, n-propyle, isopropyle, n-butyle, isobutyle, sec.-butyle, tertio-butyle, n-pentyle ou isopentyle,

X est de l'oxygène et

Y est de l'oxygène.

**4.** Procédé de production de 5-alkoxy-1,2,4-triazole-3-(thi)ones de formule (I) suivant la revendication 1

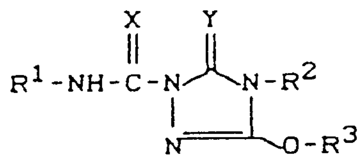

(I)

dans laquelle

$R^1$, $R^2$, $R^3$, X et Y ont les définitions indiquées dans la revendication 1,

caractérisé en ce que

(a) on fait réagir des 5-alkoxy-1,2,4-triazole-3-(thi)ones de formule générale (II)

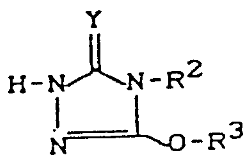

(II)

dans laquelle

$R^2$, $R^3$ et Y ont la définition indiquée ci-dessus,

avec des iso(thio)cyanates de formule générale (III)

$R^1\text{-}N=C=X$ (III)

dans laquelle

$R^1$ et X ont la définition indiquée ci-dessus, le cas échéant en présence d'un diluant et en la présence éventuelle d'un auxiliaire basique de réaction, ou bien

(b) on fait réagir des 5-alkoxy-1,2,4-triazole-3-(thi)ones de formule générale (II)

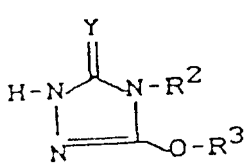

(II)

dans laquelle

$R^2$, $R^3$ et Y ont la définition indiquée ci-dessus,

avec des (thio-)carbamates réactifs de formule générale (IV)

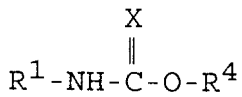
(IV)

dans laquelle

$R^1$ et X ont la définition indiquée ci-dessus et

$R^4$ est un groupe alkyle, aralkyle ou aryle,

le cas échéant en présence d'un diluant et en la présence éventuelle d'un auxiliaire basique de réaction,

(c) on fait réagir des dérivés de 5-alkoxy-1,2,4-triazole-3-(thi)ones de formule générale (V)

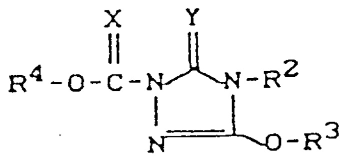
(V)

dans laquelle

$R^2$, $R^3$, X et Y ont la définition indiquée ci-dessus et

$R^4$ est un groupe alkyle, aralkyle ou aryle,

avec des composés aminés de formule générale (VI)

$R^1$-$NH_2$ (VI)

dans laquelle

$R^1$ a la définition indiquée ci-dessus,

le cas échéant en présence d'un diluant et en la présence éventuelle d'un auxiliaire basique de réaction.

5. Compositions herbicides, caractérisées par une teneur en au moins un dérivé substitué de 5-alkoxy-1,2,4-triazole-3-(thi)one de formule (I) suivant les revendications 1 à 4.

6. Procédé pour combattre des plantes indésirables, caractérisé en ce qu'on fait agir des 5-alkoxy-1,2,4-triazole-3-(thi)ones substituées de formule (I) suivant les revendications 1 à 4 sur des plantes non désirées et/ou sur leur milieu.

7. Utilisation de 5-alkoxy-1,2,4-triazole-3-(thi)ones substituées de formule (I) suivant les revendications 1 à 4 pour combattre les plantes indésirables.

8. Procédé de préparation de compositions herbicides, caractérisé en ce qu'on mélange des 5-alkoxy-1,2,4-triazole-3-(thi)ones substituées de formule (I) suivant les revendications 1 à 4 avec des diluants et/ou des substances tensio-actives.

9. 5-alkoxy-1,2,4-triazole-3-ones de formule générale (II) (Y = O)

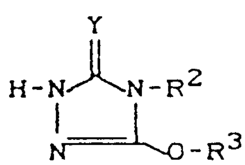
(II)

dans laquelle

$R^2$ est un groupe, linéaire ou ramifié, alkyle ayant 1 à 8 atomes de carbone, alcényle ayant 2 à 8

atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, cyanalkyle ayant 1 à 8 atomes de carbone, un groupe cycloalkylalkyle ou cycloalkyle ayant chacun 3 à 7 atomes de carbone dans la partie cycloalkyle et le cas échéant 1 à 6 atomes de carbone dans la partie alkyle linéaire ou ramifiée,

$R^3$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halgénalcynyle ayant 2 à 8 atomes de carbone et 1 à 13 atomes d'halogènes identiques ou différents, alkoxyalkyle ayant 1 à 6 atomes de carbone dans chacune des parties alkyle individuelles ou cyanalkyle ayant 1 à 8 atomes de carbone, à l'exception du composé 5-méthoxy-4-méthyl-2,4-dihydro-3H-1,2,4-triazole-3-one.

**10.** Procédé de production de 5-alkoxy-1,2,4-triazole-3-ones de formule générale (II) (Y = O)

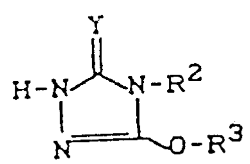

(II)

dans laquelle

$R^2$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, cyanalkyle ayant 1 à 8 atomes de carbone, un groupe cycloalkylalkyle ou cycloalkyle ayant chacun 3 à 7 atomes de carbone dans la partie cycloalkyle et le bas échéant 1 à 6 atomes de carbone dans la partie alkyle linéaire ou ramifiée,

$R^3$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halgénalcynyle ayant 2 à 8 atomes de carbone et 1 à 13 atomes d'halogènes identiques ou différents, alkoxyalkyle ayant 1 à 6 atomes de carbone dans chacune des parties alkyle individuelles ou cyanalkyle ayant 1 à 8 atomes de carbone, à l'exception du composé 5-méthoxy-4-méthyl-2,4-dihydro-3H-1,2,4-triazole-3-one,

caractérisé en ce qu'on obtient les 5-alkoxy-1,2,4-triazole-3-ones (II, Y = O) nouvelles en faisant réagir des esters d'acide hydrazino formique de formule générale (VII)

$H_2N\text{-}NH\text{-}CO\text{-}O\text{-}R^4$ (VII)

dans laquelle

$R^4$ est un groupe alkyle, aralkyle ou aryle,

avec des diesters d'acides alkylimino-carboniques de formule générale (VIII)

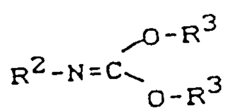

(VIII)

dans laquelle
$R^2$ et $R^3$ ont la définition indiquée ci-dessus,
le cas échéant en présence d'un diluant, à des températures comprises entre 0 °C et 200 °C.

**Revendications pour l'Etat contractant suivant : ES**

1. Procédé de production de 5-alkoxy-1,2,4-triazole-3-(thi)ones substituées de formule (I)

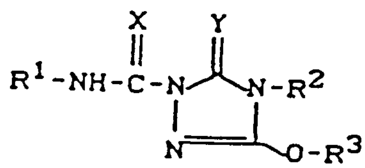

(I)

dans laquelle

$R^1$ est un groupe, linéaire ou ramifié, alkyle ayant 1 à 18 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ou halogénalcynyle ayant chacun 2 à 8 atomes de carbone et 1 à 15 et respectivement 13 atomes d'halogènes identiques ou différents, cyanalkyle ayant 1 à 8 atomes de carbone, hydroxyalkyle ayant 1 à 8 atomes de carbone et 1 à 6 groupes hydroxy, alkoxyalkyle, alkoxyminoalkyle, alkoxycarbonylalkyle ou alkoxycarbonylalcényle ayant chacun jusqu'à 6 atomes de carbone dans les parties alkyle et alcényle individuelles, alkylaminoalkyle ou dialkylaminoalkyle ayant chacun 1 à 6 atomes de carbone dans les parties alkyle individuelles ou un groupe cycloalkyle, cycloalkylalkyle, cycloalcényle ou cycloalcénylalkyle ayant chacun 3 à 8 atomes de carbone dans la partie cycloalkyle ou cycloalcényle et le cas échéant 1 à 6 atomes de carbone dans la partie alkyle et portant chacun le cas échéant un ou plusieurs substituants identiques ou différents, en choisissant comme substituants dans la partie cyclique et le cas échéant dans la partie aliphatique : un halogène, un radical cyano ainsi qu'un radical alkyle ou halogénalkyle linéaire ou ramifié ayant chacun 1 à 4 atomes de carbone et le cas échéant 1 à 9 atomes d'halogènes identiques ou différents ou un radical alcanediyle ou alcènediyle divalent ayant chacun jusqu'à 4 atomes de carbone ; en outre, $R^1$ représente un groupe hétérocyclylalkyle portant le cas échéant un ou plusieurs substituants identiques ou différents dans la partie hétérocyclyle et ayant 1 à 6 atomes de carbone dans la partie alkyle linéaire ou ramifiée et 1 à 9 atomes de carbone ainsi que 1 à 3 hétéroatomes - notamment d'azote, d'oxygène et/ou de soufre - dans la partie hétérocyclyle, en choisissant comme substituants : un halogène, un radical cyano, nitro ainsi qu'un radical alkyle, alkoxy, alkylthio, halogénalkyle, halogénalkoxy, halogénalkylthio ou alkoxycarbonyle linéaire ou ramifié ayant chacun 1 à 5 atomes de carbone et le cas échéant 1 à 9 atomes d'halogènes identiques ou différents ; en outre, $R^1$ représente un groupe, linéaire ou ramifié, alkoxy ayant 1 à 8 atomes de carbone, alcényloxy ayant 2 à 8 atomes de carbone ou alcynyloxy ayant 2 à 8 atomes de carbone et enfin un groupe aralkyle, aryloxyalkyle, arylacényle, arylalcynyle, aralkyloxy, aryloxy, aroyle ou aryle ayant chacun 6 à 10 atomes de carbone dans la partie aryle et le cas échéant jusqu'à 8 atomes de carbone dans la partie alkyle, alcényle ou alcynyle et portant chacun le cas échéant un ou plusieurs substituants identiques ou différents, en considérant comme substituants de la partie aryle : un halogène, un radical cyano, nitro, hydroxy, un radical alkyle, alkoxy, alkylthio, halogénalkyle, halogénalkoxy, halogénalkylthio, alkylsulfinyle, alkylsulfonyle, halogénalkylsulfinyle, halogénalkylsulfonyle, alcanoyle ou alkoxycarbonyle linéaire ou ramifié ayant chacun 1 à 6 atomes de carbone et le cas échéant 1 à 9 atomes d'halogènes identiques ou différents, un radical cycloalkyle ayant 3 à 6 atomes de carbone et un radical phénoxy, et en considérant le cas échéant comme substituants de la partie alkyle : un halogène ou un radical cyano,

$R^2$ représente un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou

différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, cyanalkyle ayant 1 à 8 atomes de carbone, un groupe cycloalkylalkyle ou cycloalkyle ayant chacun 3 à 7 atomes de carbone dans la partie cycloalkyle et le cas échéant 1 à 6 atomes de carbone dans la partie alkyle linéaire ou ramifiée,

$R^3$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ayant 1 à 6 atomes de carbone dans chacune des parties alkyle individuelles ou cyanalkyle ayant 1 à 8 atomes de carbone,

X représente de l'oxygène ou du soufre et

Y est de l'oxygène ou du soufre et l'halogène dans les modèles de substitution mentionnés dans $R^1$, $R^2$ et $R^3$ représente du fluor, du chlore, du brome et de l'iode.

caractérisé en ce que

(a) on fait réagir des 5-alkoxy-1,2,4-triazole-3-(thi)ones de formule générale (II)

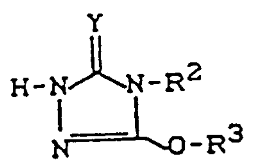

(II)

dans laquelle

$R^2$, $R^3$ et Y ont la définition indiquée ci-dessus,

avec des iso(thio)cyanates de formule générale (III)

$R^1$-N=C=X (III)

dans laquelle

$R^1$ et X ont la définition indiquée ci-dessus,

le cas échéant en présence d'un diluant et en la présence éventuelle d'un auxiliaire basique de réaction, ou bien

(b) on fait réagir des 5-alkoxy-1,2,4-triazole-(3-(thi)ones de formule générale (II)

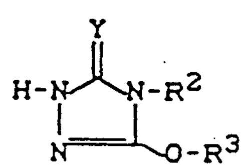

(II)

dans laquelle

$R^2$, $R^3$ et Y ont la définition indiquée ci-dessus,

avec des (thio-)carbamates réactifs de formule générale (IV)

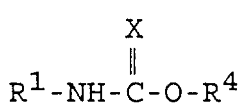

(IV)

dans laquelle

$R^1$ et X ont la définition indiquée ci-dessus et
$R^4$ est un groupe alkyle, aralkyle ou aryle,

le cas échéant en présence d'un diluant et en la présence éventuelle d'un auxiliaire basique de réaction,

(c) on fait réagir des dérivés de 5-alkoxy-1,2,4-triazole-3-(thi)ones de formule générale (V)

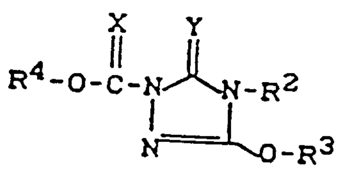

(V)

dans laquelle

$R^2$, $R^3$, X et Y ont la définition indiquée ci-dessus et
$R^4$ est un groupe alkyle, aralkyle ou aryle,

avec des composés aminés de formule générale (VI)

$R^1$-$NH_2$ (VI)

dans laquelle

$R^1$ a la définition indiquée ci-dessus,

le cas échéant en présence d'un diluant et en la présence éventuelle d'un auxiliaire basique de réaction.

**2.** Procédé de production de 5-alkoxy-1,2,4-triazole-3-(thi)ones substituées de formule générale (I) suivant la revendication 1 dans lesquelles

$R^1$ représente un groupe méthyle, éthyle, n-propyle, isopropyle, n-butyle, isobutyle, sec.-butyle, tertio-butyle, un groupe pentyle, hexyle, heptyle, octyle, nonyle, décyle ou dodécyle linéaire ou ramifié, un groupe allyle, propényle, n-butényle, isobutényle, n-pentényle, isopentényle, n-hexényle, isohexényle, propargyle, n-butynyle, isobutynyle, n-pentynyle, isopentynyle, n-hexynyle, ou isohexynyle, un groupe halogénalkyle linéaire ou ramifié ayant 1 à 4 atomes de carbone et 1 à 9 atomes d'halogènes identiques ou différents, notamment fluor, chlore ou brome, un groupe halogénalcényle ou halogénalcynyle linéaire ou ramifié ayant chacun 3 à 5 atomes de carbone et 1 à 3 atomes d'halogènes, notamment fluor ou chlore, un groupe, linéaire ou ramifié, cyanalkyle ayant 1 à 4 atomes de carbone dans la partie alkyle, hydroxyalkyle ayant 1 à 6 atomes de carbone et 1 à 3 groupes hydroxy, alkoxyalkyle, alkoxyminoalkyle, alkoxycarbonylalkyle ou alkoxycarbonylalcényle, alkylaminoalkyle ou dialkylaminoalkyle ayant chacun jusqu'à 4 atomes de carbone dans les parties alkyle et alcényle individuelles, ou un groupe cyclopropyle, cyclopropylméthyle, cyclopropyléthyle, cyclobutyle, cyclopentyle, cyclohexyle, cycloheptyle, cyclooctyle, cyclopentylméthyle, cyclohexylméthyle, cyclohexyléthyle, cyclohexényle ou cyclohexénylméthyle portant chacun le cas échéant 1 à 3 substituants identiques ou différents, en choisissant comme substituants particulièrement appréciés dans la qartie cyclique et le cas échéant dans la partie aliphatique : le fluor, le chlore, le brome, un radical méthyle, éthyle, n-propyle, isopropyle, n-butyle, isobutyle, sec.-butyle, tertio-butyle, cyano, méthanediyle, éthanediyle, butanediyle ou butadiènediyle ; en outre, $R^1$ représente un groupe hétérocyclylméthyle, hétérocyclyléthyle ou hétérocyclylpropyle portant le cas échéant dans la partie hétérocyclyle 1 à 3 substituants identiques ou différents, en considérant dans chaque cas comme hétérocycliques :

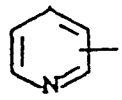 ; 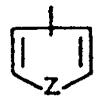 ; 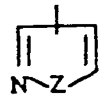 ; 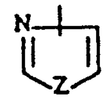 ;

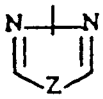 ; 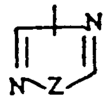 ; 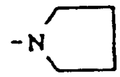 ; 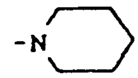 ;

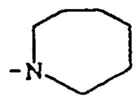 ; 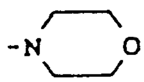 ou 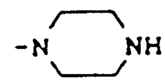

où Z represente dans chaque cas de l'oxygène ou du soufre et en choisissant comme substituants particulièrement appréciés : le fluor, le chlore, le brome, un radical cyano, nitro, méthyle, éthyle, n-propyle, isopropyle, n-butyle, isobutyle, sec.-butyle, tertio-butyle, méthoxy, éthoxy, méthylthio, trifluorométhyle, trifluorométhoxy ou trifluorométhylthio ; en outre, $R^1$ représente un groupe, linéaire ou ramifié, alkoxy ayant 1 à 6 atomes de carbone, alcényloxy ayant 3 à 6 atomes de carbone ou alcynyloxy ayant 3 à 6 atomes de carbone ou un groupe benzyle, phényléthyle, phénylpropyle, phénylbutyle, phénylpentyle, phénylhexyle, phénylheptyle, phénylcyanométhyle, phénylcyanéthyle, phénylcyanopropyle, phénoxyméthyle, phénoxyéthyle, phénoxypropyle, phénoxybutyle, phényléthynyle, benzyloxy, phényléthyloxy, phénoxy, benzoyle, phényle ou naphtyle, le cas échéant linéaire ou ramifié et portant chacun éventuellement 1 à 3 substituants identiques ou différents, en considérant comme substituants de la partie phényle : le fluor, le chlore, le brome, un radical hydroxy, cyano, nitro, méthyle, éthyle, n-propyle, isopropyle, n-butyle, isobutyle, sec.-butyle, tertio-butyle, méthoxy, éthoxy, méthylthio, trifluorométhyle, difluorométhoxy, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle et phénoxy,

$R^2$ est un groupe méthyle, éthyle, n-propyle, isopropyle, n-butyle, isobutyle, sec.-butyle, tertio-butyle, n-pentyle, isopentyle, n-hexyle ou isohexyle, un groupe allyle, propargyle, méthoxy, éthoxy, méthoxyméthyle, éthoxyméthyle, méthoxyéthyle, éthoxyéthyle, un groupe halogénalkyle, halogénalcényle ou halogénalcynyle linéaire ou ramifié ayant chacun 1 à 4 atomes de carbone et 1 à 9 atomes d'halogènes identiques ou différents, notamment fluor, chlore ou brome, un groupe cyclopentyle, cyclohexyle, cyclopropyle, cyclopropylméthyle, cyclopentylméthyle, cyclohexylméthyle ou cyclohexyléthyle,

$R^3$ est un groupe méthyle, éthyle, n-propyle, isopropyle, n-butyle, isobutyle, sec.-butyle, tertio-butyle, n-pentyle, isopentyle, sec.-pentyle, tertio-pentyle, n-hexyle ou isohexyle, un groupe allyle, propargyle, méthoxyéthyle, éthoxyéthyle, fluoréthyle, chloréthyle, fluoropropyle, chloropropyle ou cyanéthyle,

X est de l'oxygène ou du soufre et

Y est de l'oxygène ou du soufre.

**3.** Procédé de production de 5-alkoxy-1,2,4-triazoles-3-(thi)ones substituées de formule générale (I) suivant la revendication 1, dans lesquelles

$R^1$ est un groupe méthyle, éthyle, n-propyle, isopropyle, n-butyle, isobutyle, sec.-butyle, tertio-butyle, n-pentyle, isopentyle, sec.-pentyle, tertio-pentyle, n-hexyle, isohexyle, sec.-hexyle, tertio-hexyle, propargyle, n-butynyle, isobutynyle, n-pentynyle ou isopentynyle, un groupe halogénalkyle linéaire ou ramifié ayant 1 à 4 atomes de carbone et 1 à 4 atomes d'halogènes identiques ou différents, notamment fluor et chlore, un groupe cyanométhyle, cyanéthyle, n-cyanopropyle ou isocyanopropyle, un groupe cyclopropyle, cyclopropylméthyle, cyclopentyle, cyclopentylméthyle, cyclohexyle ou cyclohexylméthyle portant chacun le cas échéant 1 à 3 substituants fluoro, chloro, bromo, méthyle et/ou éthyle identiques ou différents ou un groupe phényle, benzyle, phényléthyle portant chacun le cas échéant 1 à 3 substituants fluoro, chloro, bromo, méthyle, éthyle et/ou trifluorométhyle identiques ou différents, un groupe phénylpropyle, phénylbutyle, phénylpentyle, phénylhexyle, ou morpholinyl-(alkyle en $C_1$ à

$C_4$) linéaire ou ramifié,

$R^2$ est un groupe méthyle, éthyle, n-propyle, isopropyle, cyclopropyle, n-butyle, isobutyle, sec.-butyle ou tertio-butyle,

$R^3$ est un groupe méthyle, éthyle, n-propyle, isopropyle, n-butyle, isobutyle, sec.-butyle, tertio-butyle, n-pentyle ou isopentyle,

X est de l'oxygène et

Y est de l'oxygène.

4. Compositions herbicides, caractérisées par une teneur en au moins un dérivé substitué de 5-alkoxy-1,2,4-triazole-3-(thi)one de formule (I) suivant les revendications 1 à 3.

5. Procédé pour combattre des plantes indésirables, caractérisé en ce qu'on fait agir des 5-alkoxy-1,2,4-triazole-3-(thi)ones substituées de formule (I) suivant les revendications 1 à 3 sur des plantes non désirées et/ou sur leur milieu.

6. Utilisation de 5-alkoxy-1,2,4-triazole-3-(thi)ones substituées de formule (I) suivant les revendications 1 à 3 pour combattre les plantes indésirables.

7. Procédé de préparation de compositions herbicides, caractérisé en ce qu'on mélange des 5-alkoxy-1,2,4-triazole-3-(thi)ones substituées de formule (I) suivant les revendications 1 à 3 avec des diluants et/ou des substances tensio-actives.

8. Procédé de production de 5-alkoxy-1,2,4-triazole-3-ones de formule générale (II) (Y = O)

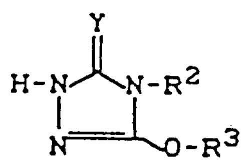

(II)

dans laquelle

$R^2$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halogénalcynyle ayant 2 à 8 atomes de carbone et 1 à 13 atomes d'halogènes identiques ou différents, alkoxyalkyle ou alkoxy ayant chacun 1 à 6 atomes de carbone dans les parties alkyle individuelles, cyanalkyle ayant 1 à 8 atomes de carbone, un groupe cycloalkylalkyle ou cycloalkyle ayant chacun 3 à 7 atomes de carbone dans la partie cycloalkyle et le cas échéant 1 à 6 atomes de carbone dans la partie alkyle linéaire ou ramifiée,

$R^3$ est un groupe, linéaire ou ramifié, alkyle ayant 1 à 8 atomes de carbone, alcényle ayant 2 à 8 atomes de carbone, alcynyle ayant 2 à 8 atomes de carbone, halogénalkyle ayant 1 à 8 atomes de carbone et 1 à 17 atomes d'halogènes identiques ou différents, halogénalcényle ayant 2 à 8 atomes de carbone et 1 à 15 atomes d'halogènes identiques ou différents, halgénalcynyle ayant 2 à 8 atomes de carbone et 1 à 13 atomes d'halogènes identiques ou différents, alkoxyalkyle ayant 1 à 6 atomes de carbone dans chacune des parties alkyle individuelles ou cyanalkyle ayant 1 à 8 atomes de carbone, à l'exception du composé 5-méthoxy-4-méthyl-2,4-dihydro-3H-1,2,4-triazole-3-one,

caractérisé en ce qu'on obtient les 5-alkoxy-1,2,4-triazole-3-ones nouvelles (II, Y = O) lorsqu'on fait réagir des esters d'acide hydrazino formique de formule générale (VII)

$H_2N\text{-}NH\text{-}CO\text{-}O\text{-}R^4$ (VII)

dans laquelle

$R^4$ est un groupe alkyle, aralkyle ou aryle

avec des diesters d'acides alkylimino-carboniques de formule générale (VIII)

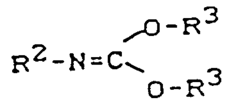

(VIII)

dans laquelle

R² et R³ ont la définition indiquée ci-dessus,

le cas échéant en présence d'un diluant, à des températures comprises entre 0 °C et 200 °C.